# EUROPEAN PATENT SPECIFICATION

(11) **EP 4 269 580 B1**
(45) Date of publication and mention of the grant of the patent: **11.03.2026**
(21) Application number: 21909917.3
(22) Date of filing: 19.10.2021
(51) Int. Cl.: C12N 15/09, C12Q 1/686, C12Q 1/6869, C12N 15/10

(54) **METHOD FOR CAUSING LARGE-SCALE DELETIONS IN GENOMIC DNA AND METHOD FOR ANALYZING GENOMIC DNA**
VERFAHREN ZUR VERURSACHUNG VON DELETIONEN IN GROSSEM MASSSTAB IN GENOMISCHER DNA UND VERFAHREN ZUR ANALYSE GENOMISCHER DNA
PROCÉDÉ POUR PROVOQUER DES DÉLÉTIONS À GRANDE ÉCHELLE DANS DE L'ADN GÉNOMIQUE ET PROCÉDÉ D'ANALYSE D'ADN GÉNOMIQUE

(30) Priority: 25.12.2020 JP 2020216271
(43) Date of publication of application: 01.11.2023
(73) Proprietor: Logomix, Inc., Tokyo 113-0023 (JP)
(72) Inventor: AIZAWA, Yasunori, Tokyo 152-8550 (JP); YAMASHITA, Hitoyoshi, Tokyo 152-8550 (JP)
(74) Representative: Hoffmann Eitle
(86) International application number: PCT/JP2021/038504
(87) International publication number: WO 2022/137760

(56) References cited:
- WO-A2-2019/023291
- CN-B- 106 637 421
- SHIYOU ZHU ET AL: "Genome-scale deletion screening of human long non-coding RNAs using a paired-guide RNA CRISPR-Cas9 library", NATURE BIOTECHNOLOGY, vol. 34, no. 12, 31 October 2016 (2016-10-31), New York, pages 1279 - 1286, XP055438119, ISSN: 1087-0156, DOI: 10.1038/nbt.3715
- KOTINI ANDRIANA G ET AL: "Engineering of targeted megabase-scale deletions in human induced pluripotent stem cells", EXPERIMENTAL HEMATOLOGY, ELSEVIER INC, US, vol. 87, 13 June 2020 (2020-06-13), pages 25 - 32, XP086232514, ISSN: 0301-472X, [retrieved on 20200613], DOI: 10.1016/J.EXPHEM.2020.06.001
- QIUPENG ZHENG, XIAOHONG CAI, MENG HOW TAN, STEVEN SCHAFFERT, CHRISTOPHER P. ARNOLD, XUE GONG, CHANG-ZHENG CHEN, SHENGLIN HUANG: "Precise gene deletion and replacement using the CRISPR/Cas9 system in human cells", BIOTECHNIQUES, INFORMA HEALTHCARE, US, vol. 57, no. 3, US , XP055548361, ISSN: 0736-6205, DOI: 10.2144/000114196
- SONG YUNING; YUAN LIN; WANG YONG; CHEN MAO; DENG JICHAO; LV QINGYAN; SUI TINGTING; LI ZHANJUN; LAI LIANGXUE: "Efficient dual sgRNA-directed large gene deletion in rabbit with CRISPR/Cas9 system", CMLS CELLULAR AND MOLECULAR LIFE SCIENCES., BIRKHAUSER VERLAG, HEIDELBERG., DE, vol. 73, no. 15, 27 January 2016 (2016-01-27), DE , pages 2959 - 2968, XP035998520, ISSN: 1420-682X, DOI: 10.1007/s00018-016-2143-z
- SRIVASTAVA VIBHA; UNDERWOOD JAMIE L.; ZHAO SHAN: "Dual-targeting by CRISPR/Cas9 for precise excision of transgenes from rice genome", PLANT CELL, TISSUE AND ORGAN CULTURE (PCTOC), SPRINGER NETHERLANDS, DORDRECHT, vol. 129, no. 1, 20 January 2017 (2017-01-20), Dordrecht, pages 153 - 160, XP036194323, ISSN: 0167-6857, DOI: 10.1007/s11240-016-1166-3

## Description

### Technical Field

The present invention as defined in the claims relates to a method for causing large-scale deletions in genomic DNA and a method for analyzing genomic DNA.

### Background Art

In higher organisms, only 1 to 2% of a genomic sequence is used to encode proteins. By contrast, in mammals, large-scale and intergenic sequences are allocated to proximal (a promoter) or distal (an enhancer, a suppressor, an insulator, etc.) regulatory elements. Thus, the selection of genes as well as the design of intergenic regions is key for genome design for functional artificial cell development. The Encyclopedia of DNA Elements (ENCODE) project suggests that a majority of intergenic regions exhibit particular transcriptional factor binding or histone modification in human cultured cells, which may possibly become a guideline for rational intergenic region design. However, it is uncertain whether all of these transcriptional and epigenetic activities are necessary for normal cell functions. Mammalian cell networks have been evolutionarily strengthened by the expansion of gene families and increase in the redundancy of gene control networks. Hence, not only nonessential genes but a majority of intergenic regions may not be necessarily required for cell survival or maintenance (Non Patent Literatures 1 to 3).
Non Patent Literature 4 relates to the genome-scale deletion screening of human long non-coding RNAs using a paired-guide RNA CRISPR-Cas9 library.
Patent Literature 1 relates to compositions and methods for making and decoding paired-guide RNA libraries and uses thereof.

### Citation List

### Non Patent Literature

Non Patent Literature 1: Xavier, J. C., Patil, K. R. & Rocha, I., Microbiol Mol Biol Rev, 78, 487-509 (2014).
Non Patent Literature 2: Posfai, G., Science, 312, 1044-1046 (2006).
Non Patent Literature 3: Hutchison, C. A. et al., Science, 351, aad6253-aad6253 (2016).
Non Patent Literature 4 : Zhu, S., et al., Nature Biotechnology, 34, 1279-1286 (2016)

### Patent Literature:

Patent Literature 1: WO 2019/023291 A2

### Summary of Invention

The present invention as defined in the claims relates to a method for causing deletions in genomic DNA and a method for analyzing genomic DNA.

The present inventors have developed a method for inducing a DNA region deletion between two target sequences using a sequence-specific nucleic acid cleaving molecule capable of sequence-specifically cleaving the target sequences. The present inventors have also found that when a DNA region to be deleted in genomic DNA is expanded, the deletion of a gene necessary for cell survival and/or proliferation negatively influences cell survival and/or proliferation. The present inventors have thereby found a method for identifying the location of a gene necessary for cell survival and/or proliferation, and this gene. Furthermore, the present inventors have found a method for preparing cells capable of surviving and/or proliferating, albeit having a DNA region deletion, by inserting a gene necessary for cell survival and/or proliferation in an ectopically expressible manner to genomic DNA. Moreover, the present disclosure provides a method for deleting a DNA region having an insert of a marker gene for negative selection. The present invention as defined in the claims is based on these findings.

The present invention is as defined in the appended claims.

### Brief Description of Drawings

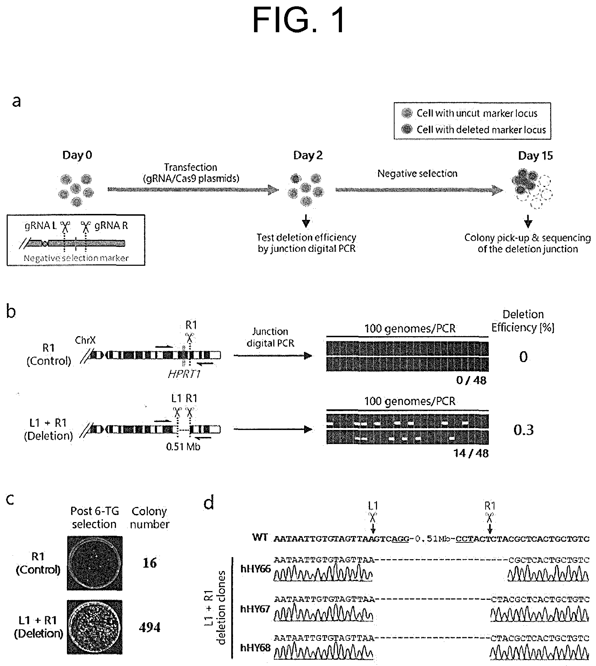

[Figure 1] Figure 1 shows a method for preparing human cells defective in a DNA region including HPRT1 gene. Panel a shows a DNA region deletion scheme using a marker gene for negative selection. The deletion of a DNA region including the marker gene for negative selection facilitates cell survival. Thus, cells having the DNA region deletion can be obtained by screening cells with the expression of the marker gene for negative selection as an index. Panel a illustrates an example using a CRISPR-Cas9 system as a sequence-specific nucleic acid cleaving molecule. Cells are transfected with a plasmid for the expression of gRNA and Cas9 endonuclease against target sequences at two locations arranged so as to flank the marker gene for negative selection. Two days later, some cells are obtained, and junction PCR of a deletion region on a genome is performed by digital PCR. The cells are cultured in a selection medium until colonies are formed. Cells having the marker gene for negative selection, i.e., cells that have not undergone the deletion, are thereby killed, whereas only cells having the deletion survive, proliferate, and form colonies. The number of colonies is counted, and junction PCR is performed again by digital PCR to confirm the presence of the deletion. Panel b shows results of deleting an endogenous HPRT1 gene locus and its neighborhood on the X chromosome, and then performing PCR of a junction region by digital PCR. In the digital PCR, 100 genomes were used as templates per PCR run, and 48 PCR runs were performed. Since amplification products were obtained in 14 cases, deletion efficiency is 14 / 48 / 100 × 100 = approximately 0.3 (%). Panel c shows colonies formed after selection with 6-thioguanine (6-TG) and the number thereof. Panel d shows the sequence of a L1-R1 deletion region and its upstream and downstream sequences. It is evident that a DNA region between two target sequences was substantially deleted.

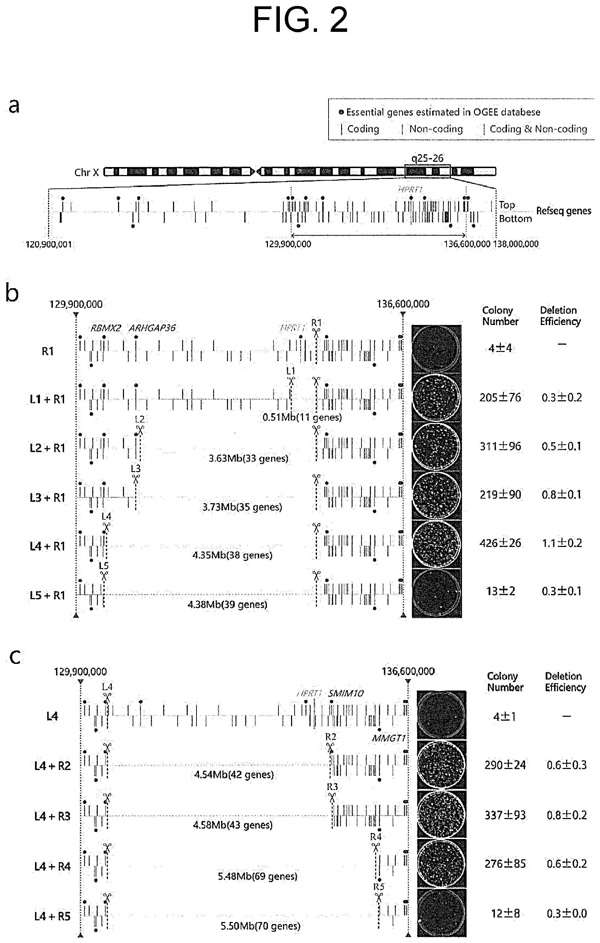

[Figure 2] Figure 2 shows the map of a genomic region around HPRT1 gene and the preparation of mega-scale deletions in the region. Panel a shows Refseq genes around the HPRT1 gene on the X chromosome. Essential genes for cell proliferation estimated from OGEE database are indicated by circles. Panels b and c show results of an experiment in which a deletion region was expanded in the centromeric direction and the telomeric direction. Target sequences of a sequence-specific nucleic acid cleaving molecule are indicated by vertical broken lines with symbols L1 to L5 and R1 to R5. The number of colonies formed after 6-TG selection of a cell population in which each DNA region deletion was induced, and deletion efficiency (%) are shown.

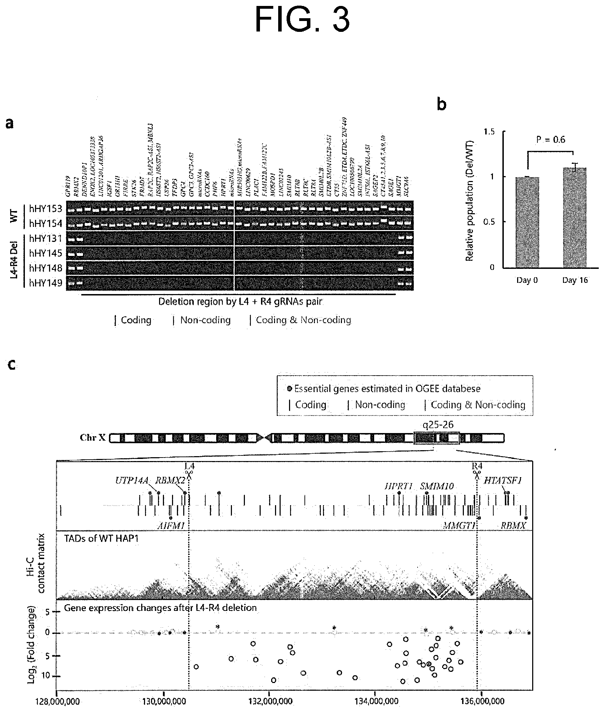

[Figure 3] Figure 3 shows the genome analysis of clones having an L4-R4 deletion. Panel a shows results of PCR to elucidate the presence of respective genes of HAP1 cells (WT) and the clones having an L4-R4 deletion. Panel b shows relative proliferative capacity of the clones having an L4-R4 deletion. The relative proliferative capacity was determined as the ratio of cells having the deletion to wild-type cells, and measured on 0 days and 16 days after deletion introduction, showing no change in the ratio. Panel c shows results of analyzing gene expression in the clones having an L4-R4 deletion using a microarray.

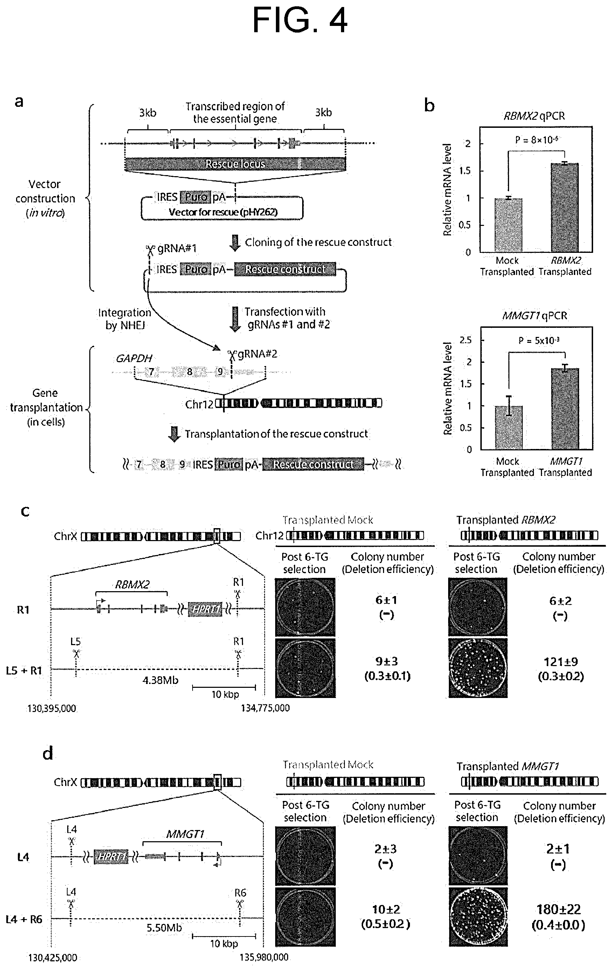

[Figure 4] Figure 4 shows mega-scale deletions including an essential gene, and the rescue experiment of the deletions. Panel a shows the scheme of gene insertion to a 3' untranslated region (UTR) of a GAPDH gene locus using pHY262 vector containing upstream and downstream 3-kb sequences flanking the essential gene. Panel b shows results of analyzing the expression of rescued RBMX2 and MMGT1 genes by real-time PCR. Panels c and d each show a line defective in endogenous RBMX2 or MMGT1 gene, and the number of colonies formed and deletion efficiency of a line in which RBMX2 or MMGT1 was rescued.

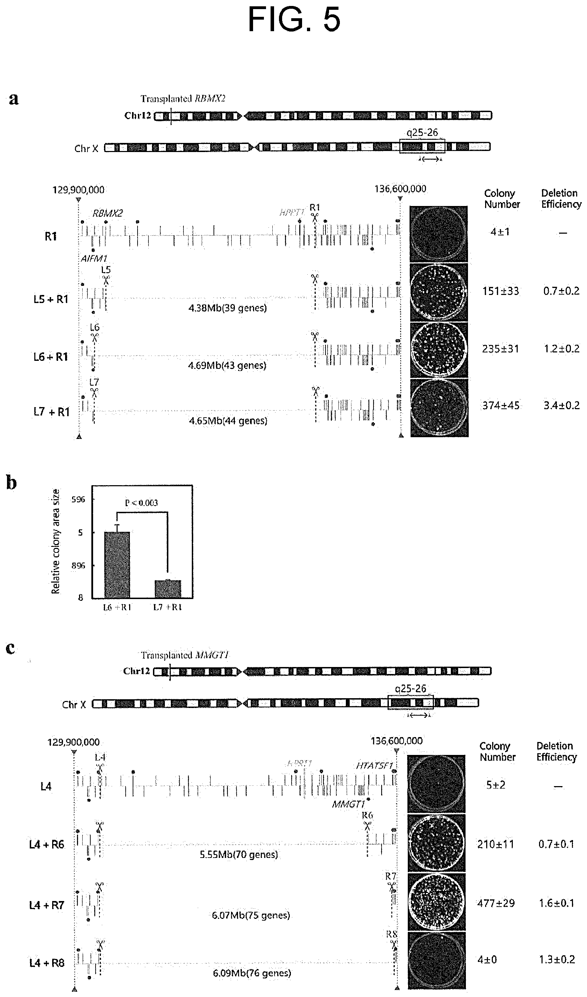

[Figure 5] Figure 5 shows results of deleting a region including an essential gene by mega-scale deletions. Panels a and c each show results of an experiment in which although a region including RBMX2 or MMGT1 gene was deleted, each gene was reintroduced to downstream of a GAPDH gene locus to further expand the deletion region. Panel b shows results of measuring a relative colony size after the experiment of panel a. The p values were determined from results of three independent experiments.

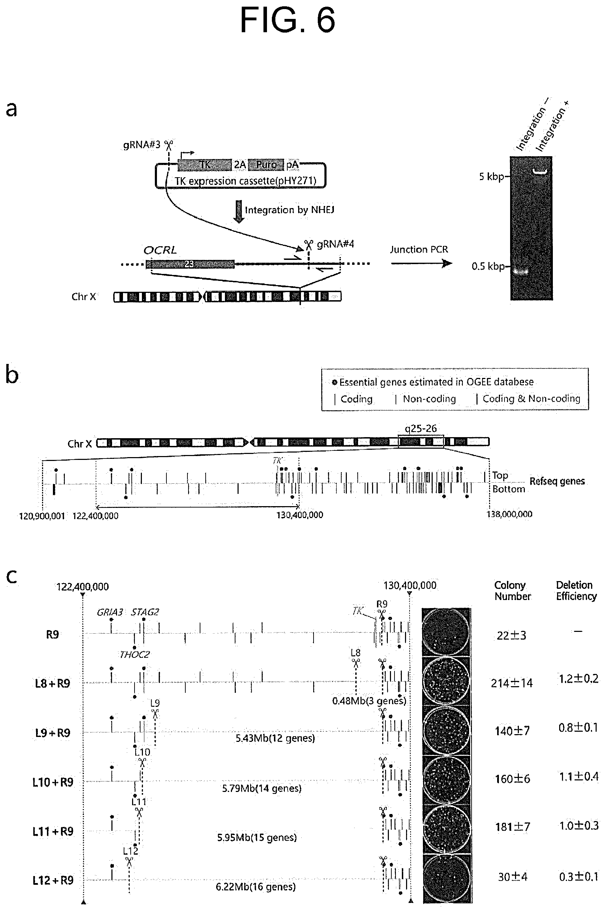

[Figure 6] Figure 6 shows the preparation of mega-scale deletions using a marker gene for negative selection in HCT116 cells. Panel a shows the scheme of thymidine kinase (TK) insertion to downstream of OCRL gene in the HCT116 cells using pHYT271 vector having a TK expression cassette. Since the size of amplification products was increased by junction PCR, the insertion was confirmed. Panel b shows the gene map of a gene locus to be deleted in the HCT116 cells. Panel c shows the manner of mega-scale DNA region deletions using TK which is a marker gene for negative selection. The deletions were expanded in the centromeric direction. The number of colonies formed after selection per deletion, and deletion efficiency are shown. The numeric values represent a mean and standard deviation from three independent experiments.

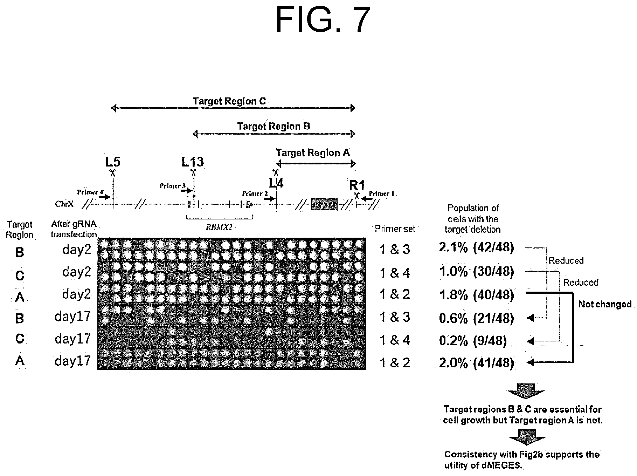

[Figure 7] Figure 7 shows a method for causing mega-scale deletions without negative selection. Three types of target regions, target regions A to C, were deleted in HAP1 cells. The presence or absence of a deletion was determined by digital PCR for 100 genomes per well 2 days and 17 days after induction of deletions in genomes, and the ratio of cells having the deletion in the cell population was determined. As a result, in the case of deleting target region B or C, the ratio of cells having the deletion in the cell population was decreased. This decrease indicates that the deleted region includes a gene necessary for cell survival and/or proliferation.

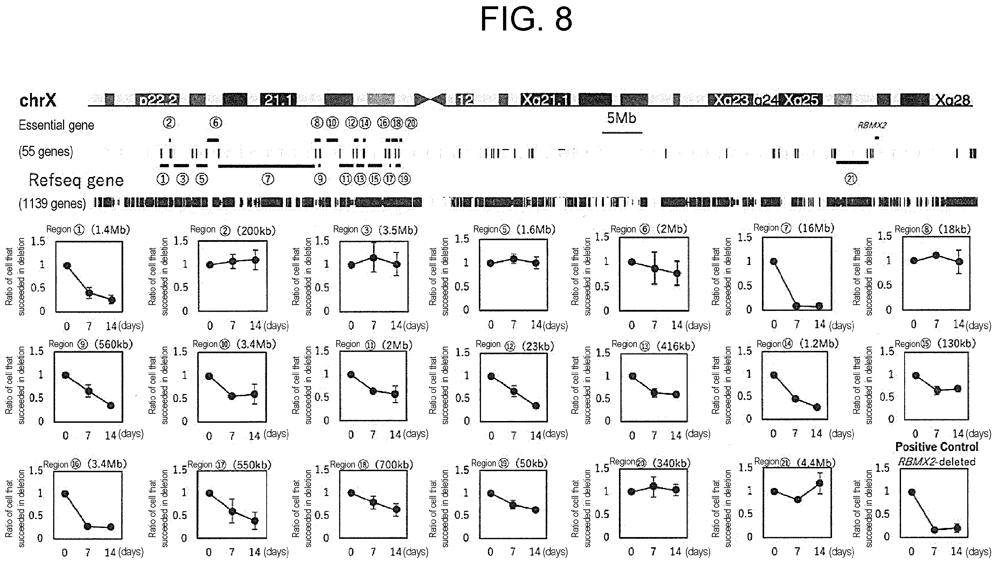

[Figure 8] Figure 8 shows results of an experiment in which each region shown in the drawing was deleted by the method of the present invention as to a region reportedly nonessential for cell survival on the human X chromosome to examine the influence of each deletion on a cell survival rate.

### Description of Embodiments

In the present specification, the term "cell" refers to a fundamental unit of life having at least genomic DNA, cytoplasm, and a membrane structure that surrounds these components. Examples of the cell include, but are not particularly limited to, cells of prokaryotes and cells of eukaryotes. The genomic DNA comprises endogenous DNA of the cell and however, is not necessarily composed of only endogenous factors of the cell.

The cell contains the genomic DNA of the cell and may further contain genomic DNA of a foreign invader (e.g., a pathogen). In the present specification, the genomic DNA of the cell itself is referred to as "host genomic DNA". The genomic DNA of an invader is capable of residing in the cell independently of the host genomic DNA and may be integrated into the host genomic DNA. The host genomic DNA may comprise a foreign factor (e.g., an insert of the whole or a portion of genomic DNA of a virus or the like).

In the present specification, the term "cell population" means a composition including a plurality of cells.

In the present specification, the term "isolation" means the separation of cells of interest from at least one of other components. The isolation can be carried out, for example, by separating and taking cells in a natural state of existence from other components existing together therewith in a natural state of existence. The isolation can be carried out, for example, by separating and taking some cells from a multicellular organism. In the present specification, a technique of handling isolated cells is referred to as an *in vitro* technique.

In the present specification, the term "purification" means the further separation of isolated cells of interest from other components existing together therewith. The purification can be carried out, for example, by separating the cells of interest from other components on the basis of morphology or a surface marker. The purification can be carried out by limiting dilution and/or cloning of cells. The purification can be carried out by establishing a cell line of the cells of interest. When the cells of interest have a marker gene such as a drug resistance gene or a gene encoding a fluorescent protein, the purification can be carried out on the basis of the expression of the marker gene. In the present specification, the term "enrich" means improvement in the existence density of the cells of interest.

In the present specification, the terms "polynucleotide" and "nucleic acid" are used interchangeably with each other and each refer to a nucleotide polymer in which nucleotides are linked through phosphodiester bonds. The "polynucleotide" or the "nucleic acid" may be DNA, may be RNA, or may be constituted by a combination of DNA and RNA. The "polynucleotide" or the "nucleic acid" may be a polymer of natural nucleotides, may be a polymer of natural nucleotides and non-natural nucleotides (analogs of natural nucleotides, nucleotides modified at one of their base moiety, sugar moiety and phosphate moiety (e.g., phosphorothioate skeletons), etc.), or may be a polymer of non-natural nucleotides.

In the present specification, the nucleotide sequence of the "polynucleotide" or the "nucleic acid" is described by generally accepted single-letter codes unless otherwise specified. Each nucleotide sequence is described from the 5' side toward the 3' side unless otherwise specified. The nucleotide residues constituting the "polynucleotide" or the "nucleic acid" may be simply described by adenine, thymine, cytosine, guanine, or uracil, etc., or their single-letter codes.

In the present specification, the term "gene" refers to a polynucleotide containing at least one open reading frame encoding a particular protein. The gene may contain both an exon and an intron.

In the present specification, the terms "polypeptide", "peptide" and "protein" are used interchangeably with each other and each refer to a polymer of amino acids linked through amide bonds. The "polypeptide", the "peptide" or the "protein" may be a polymer of natural amino acids, may be a polymer of natural amino acids and non-natural amino acids (chemical analogs, modified derivatives, etc. of natural amino acids), or may be a polymer of non-natural amino acids. Each amino acid sequence is described from the N-terminal side toward the C-terminal side unless otherwise specified.

In the present specification, the term "alleles" refer to a set of nucleotide sequences present at the same locus on the chromosomal genome. In an aspect, a diploid cell has two alleles at the same locus, and a triploid cell has three alleles at the same locus. In an aspect, an additional allele may be formed by an abnormal copy of the chromosome or an abnormal additional copy of the locus.

In the present specification, the terms "genome engineering" and "genome editing" are used interchangeably with each other and each refer to mutagenesis at a desired position (target region) in the genome. The genome engineering can involve using a sequence-specific nucleic acid cleaving molecule (e.g., sequence-specific or sequence-dependent endonuclease) designed so as to cleave DNA of the target region. In a preferred embodiment, the genome engineering can involve using nuclease manipulated so as to cleave DNA of the target region. In a preferred embodiment, the genome engineering can involve using nuclease (e.g., TALEN or zinc finger nuclease (ZFN)) manipulated so as to cleave a target sequence having a particular nucleotide sequence in the target region. In a particularly preferred embodiment, the genome engineering can involve using nuclease (e.g., a CRISPR-Cas9 system) manipulated so as to cleave a target sequence having a particular nucleotide sequence in the target region. In a preferred embodiment, the genome engineering may employ sequence-specific endonuclease such as a restriction enzyme (e.g., meganuclease) having only one cleavage site in the genome (e.g., a restriction enzyme having 16-base sequence specificity (theoretically, which is present at a ratio of 1 out of 4¹⁶ bases), a restriction enzyme having 17-base sequence specificity (theoretically, which is present at a ratio of 1 out of 4¹⁷ bases), and a restriction enzyme having 18-base sequence specificity (theoretically, which is present at a ratio of 1 out of 4¹⁸ bases)) so as to cleave a target sequence having a particular nucleotide sequence in the target region. Typically, use of site-specific nuclease induces double-strand break (DSB) in DNA of the target region, followed by the repair of the genome by an endogenous process of cells, such as homologous directed repair (HDR) and non-homologous end-joining repair (NHEJ). NHEJ is a repair method of linking ends that have undergone double-strand break, without the use of a donor DNA, and induces insertion and/or deletion (indel) with high frequency during the repair. HDR is a repair mechanism using a donor DNA and is also capable of introducing a desired mutation to a target region. Examples of the genome engineering technique preferably include a CRISPR/Cas system. The meganuclease that can be used is, for example, meganuclease selected from the group consisting of I-SceI, I-SceII, I-SceIII, I-SceIV, I-SceV, I-SceVI, I-SceVII, I-CeuI, I-CeuAIIP, I-CreI, I-CrepsbIP, I-CrepsbIIP, I-CrepsbIIIP, I-CrepsbIVP, I-TliI, I-PpoI, PI-PspI, F-SceI, F-SceII, F-SuvI, F-TevI, F-TevII, I-AmaI, I-AniI, I-ChuI, I-CmoeI, I-CpaI, I-CpaII, I-CsmI, I-CvuI, I-CvuAIP, I-DdiI, I-DdiII, I-DirI, I-DmoI, I-HmuI, I-HmuII, I-HsNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PbpIP, I-SpBetaIP, I-ScaI, I-SexIP, I-SneIP, I-SpomI, I-SpomCP, I-SpomIP, I-SpomIIP, I-SquIP, I-Ssp68031, I-SthPhiJP, I-SthPhiST3P, I-SthPhiSTe3bP, I-TdeIP, I-TevI, I-TevII, I-TevIII, I-UarAP, I-UarHGPAIP, I-UarHGPA13P, I-VinIP, I-ZbiIP, PI-Mtul, PI-MtuHIP PI-MtuHIIP, PI-PfuI, PI-PfuII, PI-PkoI, PI-PkoII, PI-Rma43812IP, PI-SpBetaIP, PI-SceI, PI-TfuI, PI-TfuII, PI-ThyI, PI-TliI, and PI-TliII, and their functional derivative restriction enzymes, or a cleavage site (or a recognition site) thereof, preferably meganuclease which is a restriction enzyme having 18-base or more sequence specificity, or a cleavage site (or a recognition site) thereof, particularly, meganuclease that does not cleave one location or two or more locations of the genome in a cell, or a cleavage site thereof.

The term "target region" refers to a region that is targeted by a genome engineering system. In the present invention, a DNA region on the genome positioned between target regions at two locations (e.g., a first target region and a second target region) can be deleted.

The term "sequence-specific nucleic acid cleaving molecule" refers to a molecule that can recognize a particular nucleic acid sequence and cleave a nucleic acid at the particular nucleic acid sequence. The sequence-specific nucleic acid cleaving molecule is a molecule having activity of cleaving a nucleic acid in a sequence-specific manner (sequence-specific nucleic acid cleaving activity).

The term "target sequence" refers to a DNA sequence, in the genome, to be cleaved by the sequence-specific nucleic acid cleaving molecule. When the sequence-specific nucleic acid cleaving molecule is Cas protein, the target sequence refers to a DNA sequence, in the genome, to be cleaved by the Cas protein. In the case of using Cas9 protein as the Cas protein, the target sequence needs to be a sequence adjacent to the 5' side of a protospacer adjacent motif (PAM). The target sequence is usually selected as a sequence of 17 to 30 bases (preferably 18 to 25 bases, more preferably 19 to 22 bases, further preferably 20 bases) immediately adjacent to the 5' side of PAM. The target sequence can be designed using a design tool known in the art such as CRISPR DESIGN (crispr.mit.edu/).

The term "Cas protein" refers to CRISPR-associated protein. In a preferred aspect, the Cas protein forms a complex with guide RNA and exhibits endonuclease activity or nickase activity. Examples of the Cas protein include, but are not particularly limited to, Cas9 protein. The Cas protein encompasses wild-type Cas protein and its homologs (paralogs and orthologs), and their mutants as long as they exhibit endonuclease activity or nickase activity in cooperation with guide RNA.

In a preferred aspect, the Cas protein is involved in a class 2 CRISPR/Cas system and more preferably involved in a type II CRISPR/Cas system. Preferred examples of the Cas protein include Cas9 protein.

The term "Cas9 protein" refers to Cas protein that is involved in a type II CRISPR/Cas system. The Cas9 protein forms a complex with guide RNA and exhibits activity of cleaving DNA of a target region in cooperation with the guide RNA. The Cas9 protein encompasses wild-type Cas9 protein and its homologs (paralogs and orthologs), and their mutants as long as they exhibit the activity described above. The wild-type Cas9 protein has a RuvC domain and a HNH domain as nuclease domains. In the present specification, any one of the RuvC domain and the HNH domain in the Cas9 protein may be inactivated. Cas9 in which any one of the RuvC domain and the HNH domain is inactivated introduces single-strand cleavage (nick) in double-stranded DNA. Hence, in the case of using Cas9 in which any one of the RuvC domain and the HNH domain is inactivated in the cleavage of double-stranded DNA, an engineering system can be configured such that a target sequence of Cas9 is set in each of the sense strand and the antisense strand and nick for the sense strand and nick for the antisense strand occur at sufficiently close positions, thereby inducing double-strand cleavage.

Examples of the organism species from which the Cas9 protein is derived preferably include, but are not particularly limited to, bacteria belonging to the genus *Streptococcus,* the genus *Staphylococcus,* the genus *Neisseria,* or the genus *Treponema.* More specifically, examples thereof preferably include Cas9 protein derived from *S. pyogenes, S. thermophilus, S. aureus, N. meningitidis,* or *T. denticola.* In a preferred aspect, the Cas9 protein is *S.* pyogenes-derived Cas9 protein.

The terms "guide RNA" and "gRNA" are used interchangeably with each other and each refer to RNA that can form a complex with Cas protein and lead the Cas protein to a target region. In a preferred aspect, the guide RNA comprises CRISPR RNA (crRNA) and transactivating CRISPR RNA (tracrRNA). crRNA is involved in binding to a target region in the genome, and tracrRNA is involved in binding to the Cas protein. In a preferred aspect, crRNA comprises a spacer sequence and a repeat sequence, and the spacer sequence binds to a complementary strand of a target sequence in the target region. In a preferred aspect, tracrRNA comprises an anti-repeat sequence and a 3' tail sequence. The anti-repeat sequence has a sequence complementary to the repeat sequence of crRNA and forms base pairs with the repeat sequence. The 3' tail sequence usually forms three stem loops.

The guide RNA may be single-guide RNA (sgRNA) in which the 5' end of tracrRNA is linked to the 3' end of crRNA, or may be formed by the base pairing of the repeat sequence and the anti-repeat sequence of crRNA and tracrRNA prepared as separate RNA molecules. In a preferred aspect, the guide RNA is sgRNA.

The repeat sequence of crRNA and the sequence of tracrRNA can be appropriately selected according to the type of the Cas protein, and sequences derived from the same bacterial species as that for the Cas protein can be used. S. pyogenes-derived Cas9 protein, crRNA, and tracrRNA (or sgRNA) can be used for a CRISPR-Cas9 system. Various crRNA repeat sequences and tracrRNA sequences for sgRNA design have been proposed. Those skilled in the art can design sgRNA on the basis of a technique known in the art (e.g., Jinek et al. (2012) Science, 337, 816-21; Mali et al. (2013) Science, 339: 6121, 823-6; Cong et al. (2013) Science, 339: 6121, 819-23; Hwang et al. (2013) Nat. Biotechnol. 31: 3, 227-9; Jinek et al. (2013) eLife, 2, e00471).

The term "operably linked" used in relation to a polynucleotide means that a first nucleotide sequence is placed sufficiently close to a second nucleotide sequence so that the first nucleotide sequence is capable of influencing the second nucleotide sequence or a region controlled by the second nucleotide sequence. For example, the phrase "polynucleotide is functionally linked to a promoter" means that the polynucleotide is linked so as to be expressed under the control of the promoter.

The term "expressible state" refers to a state in which a polynucleotide can be transcribed in a cell harboring the polynucleotide.

The term "expression vector" is a vector containing a subject polynucleotide and refers to a vector having a system that puts the subject polynucleotide in an expressible state in a cell harboring the vector. For example, the "Cas protein expression vector" means a vector that permits expression of the Cas protein in a cell harboring the vector. For example, the "guide RNA expression vector" means a vector that permits expression of the guide RNA in a cell harboring the vector.

In the present specification, the sequence identity (or homology) between nucleotide sequences or amino acid sequences is determined as the ratio of identical bases or amino acids to the whole nucleotide sequences or the whole amino acid sequences, except for gaps, in alignments obtained by juxtaposing two nucleotide sequences or amino acid sequences so as to attain the highest identity of the corresponding bases or amino acids while placing the gaps in moieties corresponding to insertion and deletion. The sequence identity between nucleotide sequences or amino acid sequences can be determined using various homology search software known in the art. For example, the value of sequence identity between nucleotide sequences can be obtained by calculation based on alignments obtained with homology search software BLASTN known in the art, and the value of sequence identity between amino acid sequences can be obtained by calculation based on alignments obtained with homology search software BLASTP known in the art.

### <Method of present invention as defined by the claims>

The method of the present invention as defined by the claims can be an *in vitro* method. The method of the present invention as defined by the claims can also preferably employ isolated cells.

Thus, the present invention provides an *in vitro* method comprising:
(a) providing a cell population comprising isolated cells; and
(b) allowing a sequence-specific nucleic acid cleaving molecule capable of sequence-specifically cleaving target sequences at two locations on genomic DNA to act on genomic DNA of cells in the cell population so that cleavage occurs in each of the target sequences at two locations on the genomic DNA, thereby causing a DNA region deletion in a region between the cleavage sites at two locations in at least some cells in the cell population,
(c) determining a ratio of cells having the DNA region deletion to the total cells in the cell population comprising the cells having the DNA region deletion as defined in the claims.

In the (a), the cells contained in the cell population may be of a single type or may be of plural types. Preferably, the cells contained in the cell population can be of a single type (e.g., a cell line or cloned cells). The cell population may be a population of floating cells or may be a population of adherent cells. The cell population may be a population of cells prepared as single cells or may be a cell population comprising cell masses. The cell population can comprise the cells and a physiologically acceptable excipient. The physiologically acceptable excipient is an excipient having conditions suitable for the maintenance of the cells. Examples thereof include water, salts, pH buffers, and isotonic agents. The cell population can comprise, for example, 10² or more, 10³ or more, 10⁴ or more, 10⁵ or more, or 10⁶ or more cells.

In an aspect of the present invention as defined by the claims, the cells can be isolated cells. The cells may be purified cells. The cells can be cells of a unicellular organism. The cells can be a cell line. The cells can be cloned cells (cell clones). The cells can be cells prepared as single cells. The cells can be floating cells. The cells can be adherent cells. The cells may form cell aggregates. The cells may form colonies. The cells are placed under conditions suitable for the maintenance or proliferation thereof.

In an aspect, the cells can be cells selected from the group consisting of pluripotent cells and pluripotent stem cells (embryonic stem cells and induced pluripotent stem cells, etc.). In an aspect, the cells can be tissue stem cells. In an aspect, the cells can be somatic cells. In an aspect, the cells can be tissue progenitor cells. In an aspect, the cells can be germline cells (e.g., germ cells). In an aspect, the cells can be a cell line. In an aspect, the cells can be immortalized cells. In an aspect, the cells can be cancer cells. In an aspect, the cells can be noncancer cells. In an aspect, the cells can be cells of a disease patient. In an aspect, the cells can be cells of a healthy individual. In an aspect, the cells can be cells infected with a foreign pathogen. In an aspect, the cells can be noninfected cells. In an aspect, the cells can be animal cells (for example, bird cells, for example, fish cells, for example, amphibian cells, for example, reptilian cells, for example, mammalian cells, for example, rodent cells, for example, primate cells, and, for example, human cells). In an aspect, the cells can be cells (particularly, recombinant protein-producing cells) selected from the group consisting of, for example, insect cells (e.g., silkworm cells), HEK293 cells, HEK293T cells, Expi293F(TM) cells, FreeStyle(TM) 293F cells, Chinese hamster ovary cells (CHO cells), CHO-S cells, CHO-K1 cells, and ExpiCHO cells, and derivative cells from these cells. In an aspect, the cells can be plant cells. In an aspect, examples of the cells also include microbial cells, for example, cells of microbes including gram positive bacteria such as filamentous bacteria and *Actinomyces,* gram negative bacteria such as *E. coli,* and fungi such as yeasts.

The cells used in the present invention may be preferably haploid cells or diploid cells. The cells may be other polyploid cells, which can be used without particular limitations. In every embodiment of the present invention, haploid cells can be preferably used.

In the (b), a sequence-specific nucleic acid cleaving molecule capable of sequence-specifically cleaving target sequences at two locations on genomic DNA can be allowed to act on genomic DNA (particularly, host genomic DNA) of cells. The target sequences can be appropriately set by those skilled in the art in light of the cleavage characteristics of the sequence-specific nucleic acid cleaving molecule used. The target sequences reside in the nucleotide sequence of at least one allele and can be sequences that reside in common in preferably two or more or all alleles. For cleaving a plurality of target sequences on a genome, for example, any of a CRISPR-Cas9 system, TALEN, and zinc finger nuclease can be preferably used. This is specifically because a plurality of locations can be cleaved by merely increasing the number of guide RNA (a combination of crRNA and tracrRNA, or sgRNA) with increase in the number of target sequences (e.g., see WO2014/093661). In the case of using a genome engineering system other than the CRISPR-Cas9 system in cleavage, cleaving molecules are provided on a target sequence basis.

When cleavage occurs in genomic DNA (particularly, host genomic DNA), the cleavage site is joined again through the genomic repair mechanism of cells. When cleavage occurs at two locations in genomic DNA, the cleaved end on the telomeric side and the cleaved end on the centromeric side in the genomic DNA are directly joined at a given probability through the genomic repair mechanism so that a DNA region between the cleavage sites at two locations can be deleted from the genomic DNA.

Thus, in the (b), a cell population comprising at least one cell (preferably a plurality of cells) having the DNA region deletion that has occurred in the region between the cleavage sites at two locations is obtained.

In the (b), the cleavage sites at two locations can be designed so as to flank a region including one or more genes or gene candidates. The one or more genes or gene candidates included in the cleavage sites at two locations may comprise one or more genes that positively control cell survival and/or proliferation (e.g., genes essential for cell survival and/or proliferation). The one or more genes or gene candidates included in the cleavage sites at two locations may comprise one or more genes that promote cell proliferation when deleted, such as one or more genes that suppress cell proliferation. The genes that positively control cell survival and/or proliferation (e.g., genes essential for cell survival and/or proliferation) are not necessarily required to be grasped in advance by those skilled in the art. However, the genes that positively control cell survival and/or proliferation (e.g., genes essential for cell survival and/or proliferation) can be predicted in advance using a published database such as Online Gene Essentiality (OGEE) database (http://ogee.medgenius.info/browse/).

In an aspect, the region including one or more genes or gene candidates may include a gene predicted as a gene that positively controls cell survival and/or proliferation (e.g., a gene essential for cell survival and/or proliferation). In an aspect, the region including one or more genes or gene candidates may include a gene that negatively controls cell survival and/or proliferation or a gene predicted as the gene. In an aspect, the region including one or more genes or gene candidates may include a gene that positively controls cell survival and/or proliferation (e.g., a gene essential for cell survival and/or proliferation) or a gene predicted as the gene, and a gene that negatively controls cell survival and/or proliferation or a gene predicted as the gene.

In the (b), the cleavage sites at two locations are not particularly limited and may be designed so as to flank a region of 0.1 Mb or more, 0.2 Mb or more, 0.3 Mb or more, 0.4 Mb or more, 0.5 Mb or more, 0.6 Mb or more, 0.7 Mb or more, 0.8 Mb or more, 0.9 Mb or more, 1 Mb or more, 2 Mb or more, 3 Mb or more, 4 Mb or more, or 5 Mb or more. Specifically, the DNA region to be deleted from the genomic DNA may have a length of 0.1 Mb or more, 0.2 Mb or more, 0.3 Mb or more, 0.4 Mb or more, 0.5 Mb or more, 0.6 Mb or more, 0.7 Mb or more, 0.8 Mb or more, 0.9 Mb or more, 1 Mb or more, 2 Mb or more, 3 Mb or more, 4 Mb or more, or 5 Mb or more.

In the (b), a cell population comprising cells having the DNA region deletion that has occurred in a region between the cleavage sites at two locations can thereby be obtained. While DNA, such as genomic DNA, having cleavage at two locations is being capable of repaired in cells, this repair can result in cells having DNA, such as genomic DNA, having the DNA region deletion that has occurred in a region between the cleavage sites at two locations with a given probability. Thus, the cell population comprising cells having the DNA region deletion that has occurred in a region between the cleavage sites at two locations can be obtained as described above.

The cells having the DNA region deletion that has occurred in a region between the cleavage sites at two locations can be obtained from the cell population. The obtainment can be performed, for example, by the limiting dilution and cloning of the cell population. In the genomic DNA from which the DNA region has been deleted, it is considered that portions upstream and downstream of the deleted site are directly joined. Thus, primers for amplifications are designed so as to flank the junction, and the presence or absence of the DNA region deletion can be determined by PCR using, as a template, the genomic DNA from which the DNA region has been deleted (junction PCR). Also, the presence or absence of the DNA region deletion can be determined by sequencing the junction.

In this respect, when the deleted DNA region includes no gene that positively controls cell survival and/or proliferation (e.g., gene essential for cell survival and/or proliferation), cells having the DNA region deletion can be obtained. When the deleted DNA region includes a gene that positively controls cell survival and/or proliferation (e.g., a gene essential for cell survival and/or proliferation), cells having the deletion arrest proliferation or are killed with time, decreasing the ratio of the cells occupying the cell population. Alternatively, when the deleted DNA region includes a gene that negatively controls cell survival and/or proliferation, cells having the deletion proliferate with time, increasing the ratio of the cells occupying the cell population.

In the (c), the influence of the DNA region deletion on cell proliferation or survival can be determined on the basis of the ratio of cells having the DNA region deletion.

The influence of the DNA region deletion on cell proliferation or survival can be determined by various methods.

The (c) can be performed subsequently to the step (b). In this context, it is possible that cell screening (isolation of particular cells from other cells, for example, screening for some cells from the cell population) is not carried out between the steps (b) and (c). Specifically, the step (c) can be carried out by culturing, as it is, the cell population obtained in the step (b). The culture can be performed under conditions suitable for the culture (or proliferation) of the cells before deletion introduction. Alternatively, a cell screening step may be carried out between the steps (b) and (c).

In the (c), the determination can be performed by: determining DNA region deletion efficiency or an estimate thereof; and then comparing a ratio of cells having the DNA region deletion in the cell population after culture with the determined deletion efficiency or estimate thereof.

In the (c), the deletion efficiency or the estimate thereof, and the ratio of cells having the DNA region deletion after culture can each be determined as a ratio of cells having the deletion to the total cells contained in a suspension containing the cell population. The ratio can be determined by a counting technique of genomic DNA having the deletion and genomic DNA having no deletion, contained in the suspension.

The influence of the DNA region deletion on cell proliferation or survival can be determined from deletion introduction efficiency (or an estimate thereof) and the ratio of cells having the DNA region deletion after subsequent culture. The (c) {hereinafter, the same holds true for the step of determining the influence of the DNA region deletion} can comprise, for example, calculating deletion introduction efficiency (or an estimate thereof). The DNA region deletion introduction efficiency can be determined after treatment of the (b) and before exertion of an effect of the DNA region deletion on cells. Immediately after introduction of the DNA region deletion, the influence of the DNA region deletion is small because a transcription product or a translation product derived from the deleted DNA region remains in cells. Thus, the DNA region deletion introduction efficiency can be confirmed 2 hours to 3 days, 4 hours to 3 days, 6 hours to 3 days, 8 hours to 3 days, 12 hours to 3 days, 18 hours to 3 days, 1 day to 2 days, 1 day to 3 days, 1 day to 60 hours, 4 hours to 60 hours, 4 hours to 48 hours, 4 hours to 36 hours, 4 hours to 30 hours, or 4 hours to 2 days after treatment of the (b). The (c) {hereinafter, the same holds true for the step of determining the influence of the DNA region deletion} can also comprise culturing the obtained cells. The culture can be performed under conditions suitable for the culture of cells before DNA region deletion treatment. For example, the cell population comprising the cells having the DNA region deletion, obtained after the (b) can be cultured and evaluated by counting the number of cells that have proliferated per given number of cells, or the number of colonies formed by the cells that have proliferated. Specifically, change in the number of cells that have proliferated per given number of cells, or the number of colonies formed by the cells that have proliferated (or decrease in the ratio of colonies or cells having the deletion from the deletion introduction efficiency) means that the deleted DNA region includes a gene that controls cell survival and/or proliferation. More specifically, a large or increased number of cells that have proliferated per given number of cells, or number of colonies formed by the cells that have proliferated can be evaluated as the DNA region deletion positively influencing cell proliferation or survival, or can be evaluated as the DNA region including a gene that negatively controls cell survival and/or proliferation. A small or decreased number of cells that have proliferated per given number of cells, or number of colonies formed by the cells that have proliferated can be evaluated as the DNA region deletion negatively influencing cell proliferation or survival, or can be evaluated as the DNA region including a gene that positively controls cell survival and/or proliferation (particularly, a gene essential for cell survival and/or proliferation). The culture can be attained under conditions suitable for the culture of cells before engineering. The ratio of colonies having the DNA region deletion to the number of colonies formed may be examined.

The deletion introduction efficiency (or the estimate thereof) and the ratio of the number of cells having the DNA region deletion in the cell population may be determined, for example, by use of a counting approach such as digital PCR or a digital counting technique of nucleic acids with a molecular barcode. The counting approach does not have to involve separating certain cells from other cells, and/or allowing cells to form colonies. Specifically, the deletion introduction efficiency (or the estimate thereof) and the ratio of the number of cells having the DNA region deletion in the cell population can be calculated using a cell suspension (or using genomic DNA extracted from the cell suspension or amplification products thereof).

The digital PCR is an approach of dispensing nucleic acids (here, genomic DNA) in a concentration and an amount that attain a constant number of genomes per well to a plurality of fine divisions, subjecting the nucleic acids to PCR reaction, and counting the number of wells in which PCR reaction has occurred, thereby performing the absolute quantification of the template nucleic acids. The nucleic acids can be distributed into a plurality of fractions each containing one molecule by use of a microfluidic device or a droplet method using water-in-oil droplets, and nucleic acid amplification reaction is caused in parallel in the fractions each containing one molecule. No amplification product is formed in the absence of the template to be amplified, whereas amplification products are formed in the presence of the template to be amplified. By use of this, the presence of the template to be amplified in a sample or the abundance thereof can be digitally determined by counting the presence or absence of amplification on a fraction basis. PCR primers can be designed so as to increase when the DNA region deletion does not occur and so as not to increase in the presence of the deletion, or can be designed so as not to increase when the DNA region deletion does not occur and so as to increase in the presence of the deletion.

The digital counting technique of nucleic acids with a molecular barcode is a technique of adding a unique molecular barcode (specifically, a unique nucleotide sequence) per molecule to nucleic acid fragments, and then sequencing the sequences thereof, thereby absolutely quantifying the number of nucleic acid molecules. In the digital counting technique of nucleic acids with a molecular barcode, the number of types of molecular barcodes corresponds to the number of nucleic acid molecules.

When the DNA region includes a gene that positively controls cell survival and/or proliferation (particularly, a gene essential for cell survival and/or proliferation), the cells are killed as cultured. Thus, the influence of the DNA region deletion on cell proliferation or survival can be evaluated, for example, by determining the ratio of the number of cells having the DNA region deletion in the cell population before and after this killing (or decrease in the ratio of colonies or cells having the deletion from the deletion introduction efficiency).

The influence of the DNA region deletion on cell proliferation or survival can be determined by comparison to a control. The influence of the DNA region deletion on cell proliferation or survival can be determined, for example, by comparison to the proliferation or survival of control cells. The control cells can be, for example, cells having a portion or the whole of the DNA region.

In the present invention, the (c) {hereinafter, the same holds true for the step of determining the influence of the DNA region deletion} comprise culturing the cells obtained in the (b). The culture can be performed, for example, under predetermined conditions. Examples of the predetermined conditions include usual cell culture conditions as well as culture in the presence of stress, in the presence of stimulation of proliferation, in the presence of stimulation of induction of differentiation, under hypoxic conditions, in the presence of a growth factor, in the presence of a proliferation inhibitor, in the presence of a differentiation inducing factor, in the presence of a differentiation inhibiting factor, and in the presence of a drug. In this way, the influence (role) of the deleted DNA region on the behavior of the cells under the predetermined conditions can be determined by culturing the cells obtained in the (b) under the predetermined conditions. The influence of the culture conditions (predetermined conditions) on the behavior of cells having a deletion of a particular DNA region can also be examined. For example, a drug can be characteristically analyzed or screened for by examining the influence of addition of the drug on the behavior of cells having a deletion of a particular DNA region.

The method of the present invention may comprise the (a) to the (c) and (d) determining whether or not the deleted DNA region compared with control genomic DNA includes a gene that controls cell survival and/or proliferation on the basis of the presence or absence or a magnitude of the influence on cell survival and/or proliferation in the (c) as compared with a cell population used as a control.

In the (d), the control or a negative control can be unengineered cells or cell population or cells or a cell population before engineering. In the (d), the control (or a negative control) can be cells having a deletion of a smaller DNA region or a cell population comprising the cells. In the (d), the control (or a positive control) can be cells having a deletion of a larger DNA region or a cell population comprising the cells. In the (d), the control can be cells having a deletion of a different DNA region or a cell population comprising the cells. The cell population can be obtained by the (b).

In the (d), the influence of the gene included in the deleted DNA region on cell survival and/or proliferation can be evaluated by using unengineered cells or cell population or cells or a cell population before engineering as a control.

In the (d), the influence of a gene that is contained in a control and has been deleted by the deletion of the DNA to be evaluated on cell survival and/or proliferation can be evaluated by using, as the control, cells having a deletion of a smaller DNA region or a cell population comprising the cells.

In the (d), the influence of a gene that is not contained in a control and is contained in cells having the deletion of the DNA to be evaluated on cell survival and/or proliferation can be evaluated by using, as the control, cells having a deletion of a larger DNA region or a cell population comprising the cells.

In the (d), the influence of (d-1) a gene that is contained in a control and has been deleted by the deletion of the DNA to be evaluated and/or (d-2) a gene that is not contained in a control and is contained in cells having the deletion of the DNA to be evaluated on cell survival and/or proliferation can be evaluated by using, as the control, cells having a deletion of a different DNA region or a cell population comprising the cells.

In an aspect, the method of the present invention can comprise the (a) to the (c) and (e) further comprising determining the ratio of the cells at at least two different points in time, and determining whether or not the deleted DNA region includes at least one gene that controls cell survival and/or proliferation on the basis of the presence or absence or a magnitude of the influence on cell survival and/or proliferation in the (c) over time.

A cell population comprising cells having the DNA region deletion is obtained by the (b). The ratio of the cells having the deletion, contained in the cell population depends on genome engineering efficiency. The cells having the deletion, contained in the cell population are capable of increasing in number, decreasing in number, or maintaining their number by culture. The cells are capable of exhibiting, for a while even after genome engineering, similar behavior to that before engineering due to a remaining transcript (e.g., mRNA) from genomic DNA in cytoplasm or a remaining translation product (e.g., a protein) from the transcript. However, the amount of the transcript remaining or the amount of the protein remaining decreases by culture for a while so that a genotype ascribable to the DNA deletion is manifested as a phenotype. Then, a phenotype ascribable to the gene that controls cell survival and/or proliferation is elucidated.

In the (e), as for the two points in time (a first point in time and a second point in time), the first point in time can be before the genotype ascribable to the DNA deletion is manifested as a phenotype, and the second point in time can be after the genotype ascribable to the DNA deletion is manifested as a phenotype. The first point in time may be, for example, within 3 days from genome engineering. The second point in time may be, for example, on 3 days or later after genome engineering. The interval between the first point in time and the second point in time can be a period of, for example, 1 day or more, 2 days or more, 3 days or more, 4 days or more, 5 days or more, 6 days or more, or 1 week or more. Those skilled in the art can carry out the present invention as defined by the claims by appropriately determining the first point in time and the second point in time in light of the state of the cells, etc.

The (e) further comprises determining the ratio of the cells at at least two different points in time, and evaluating the presence or absence or a magnitude of the influence on cell survival and/or proliferation in the (c) over time.

In the (e), when the influence on cell survival and/or proliferation in the (c) over time is found, the possibility is suggested that the causative DNA region deletion thereof includes a gene that controls cell survival and/or proliferation. When the influence on cell survival and/or proliferation in the (c) over time is found, the magnitude of power of influence of the gene that controls cell survival and/or proliferation, included in the causative DNA region deletion thereof, on cell survival and/or proliferation can be evaluated by evaluating the magnitude of the influence of the DNA region deletion. The magnitude of the influence can be evaluated by comparison to a control. The control can be a positive control such as a gene encoding a cell growth factor and/or a positive control such as a gene encoding a cell proliferation inhibiting factor. A negative control such as a gene encoding a factor known to have no influence on cell survival and/or proliferation may be used as the control.

When the deleted DNA region includes a plurality of putative genes (or genes), (f) determining whether any gene thereamong is a gene that controls cell survival and/or proliferation may be carried out. The determination of whether any gene is a gene that controls cell survival and/or proliferation can be carried out by use of various methods. Whether any gene is a gene that controls cell survival and/or proliferation can be determined by, for example, specific gene disruption, subdivision of a defective region ascribable to the gene deletion according to the present invention as defined by the claims, or gene cloning and functional analysis of the gene.

When the deleted DNA region includes a gene that controls cell survival and/or proliferation, the gene can be ectopically introduced to another location of the genomic DNA. Thus, the method of the present invention may further comprise (g) ectopically introducing at least one gene that controls cell survival and/or proliferation (particularly, gene that positively controls cell survival and/or proliferation) to the genomic DNA having the DNA region deletion, the gene being operably linked to a control sequence.

In this context, the ectopic introduction means introduction to a location different from a location where the gene is endogenously positioned.

By the (b) and the (g), even while the DNA region deletion is caused in genomic DNA, the gene that controls cell survival and/or proliferation (particularly, a gene that positively controls cell survival and/or proliferation, particularly, a gene essential for cell survival) is ectopically introduced to the genomic DNA, whereby a deletion of a larger DNA region can be caused in the genomic DNA while the influence on cell survival and/or proliferation is reduced. In this way, the genomic DNA of the cells can be minimized into a state having a gene set essential for survival.

The present disclosure also provides a reference method comprising:
(α) providing a cell population comprising cells, wherein the cells comprise a negative selection marker gene in a region to be deleted;
(β) allowing a sequence-specific nucleic acid cleaving molecule capable of sequence-specifically cleaving target sequences at two locations on genomic DNA to act on genomic DNA of cells in the cell population so that cleavage occurs in each of the target sequences at two locations on the genomic DNA, thereby causing a DNA region deletion in a region between the cleavage sites at two locations in at least some cells in the cell population, wherein the two locations are designed to be at positions flanking the marker gene for negative selection; and
(y) selecting cells lacking the negative selection marker gene.
The reference method of the present disclosure can be an *in vitro* method, and the cells can be isolated cells. In an aspect, the reference method of the present disclosure is an *in vitro* method, and the cells are isolated cells.

The (α) is the same as the (a) except that the cells comprise a negative selection marker gene in a region to be deleted, so that the description about the same portion is omitted. In this context, in the case of selecting cells expressing no selection marker from a cell population in which cells expressing a selection marker coexist with the cells expressing no selection marker, the selection marker is referred to as a "negative selection marker" or a "selection marker for negative selection".

The negative selection marker is not particularly limited as long as cells that do not express this marker can be selected. Examples of the negative selection marker gene include suicide genes (thymidine kinase (TK)), etc.), fluorescent protein genes, luminescent enzyme genes, and chromogenic enzyme genes. When the negative selection marker gene is a gene that negatively influences cell survival (e.g., a suicide gene), the negative selection marker gene can be functionally linked to an inducible promoter. The functional linkage to an inducible promoter enables the negative selection marker gene to be expressed only when cells having the negative selection marker gene are desired to be removed. When the negative selection marker gene has small negative influence on cell survival, for example, when the negative selection marker gene is a marker gene optically detectable by fluorescence, luminescence, or color development, etc. (visible marker gene), the negative selection marker gene may be constitutively expressed.

In the (α), the cells comprise a negative selection marker gene (i.e., a marker gene that can be used in negative selection) in a DNA region to be deleted. The DNA region to be deleted can be set to a site endogenously having the negative selection marker gene, or can be prepared by extraneously introducing the negative selection marker gene to the DNA region to be deleted. The site endogenously having the negative selection marker gene can be, for example, a region (particularly, a q25-26 DNA region on the human X chromosome) having HPRT1 gene on the X chromosome (particularly, human HPRT1 gene on the human X chromosome). The extraneous introduction of the negative selection marker gene to the region to be deleted can be performed, for example, by use of a gene engineering technique (e.g., HDR or a genome editing system). When the negative selection marker gene is, for example, a visible marker gene, for example, the visible marker gene is inserted to a particular DNA region of genomic DNA and then expressed. Whether the visible marker gene has been introduced to one allele or whether the visible marker gene has been introduced to a plurality of alleles can thereby be determined from the luminescence intensity thereof. Cells having an insert of the visible marker gene in the particular DNA region can be cloned to obtain cells comprising the negative selection marker gene in the region to be deleted. Alternatively, a unique selection marker gene distinguishable from each other and a negative selection marker gene are inserted to each of a plurality of alleles, and cells can be selected by using the expression of the unique selection marker integrated in each of the alleles as an index to obtain cells having the negative selection marker gene integrated in a plurality of alleles. The insertion of the gene to the particular DNA region can be achieved by: cleaving a target sequence in the DNA region using a genome engineering system; and inducing HDR by donor DNA that has an upstream homology arm capable of being homologously recombined with an upstream site of a cleavage site and a downstream homology arm capable of being homologously recombined with a downstream site of the cleavage site, and comprises the gene to be inserted between the upstream homology arm and the downstream homology arm. When the negative selection marker gene is a gene that negatively influences cell survival, such as a suicide gene, cells that kill themselves by the expression of the suicide gene in each clone after cloning are cells harboring the negative selection marker gene in at least one allele. In the case of also introducing the negative selection marker gene to an additional allele, the negative selection marker gene may be functionally (operably) linked to another inducible promoter and introduced to the additional allele. It can be confirmed that cells are not killed even when the promoter is driven. Diploid cells, preferably haploid cells, can be used as the cells. For example, HAP1 cells can be used as the haploid cells from the viewpoint of the convenience of an assay system.

In the (β), target sequences at two locations are determined so as to flank the DNA region having an insert of the marker gene for negative selection in the (α), and a sequence-specific nucleic acid cleaving molecule capable of sequence-specifically cleaving the target sequences can be designed. When the sequence-specific nucleic acid cleaving molecule is allowed to act on genomic DNA, the DNA region between the target sequences at two locations is deleted from the genomic DNA. Since the DNA region between the target sequences at two locations includes the marker gene for negative selection, the marker gene for negative selection is also deleted together therewith by the deletion.

In the (y), cells having the DNA region deletion between the target sequences at two locations, obtained by the (β) are selected. The cells having the DNA region deletion between the target sequences at two locations lack the marker gene for negative selection, as mentioned above. Thus, the cells, when maintained under conditions that induce the marker gene for negative selection, do not express the marker gene for negative selection. Thus, the cells having the DNA region deletion between the target sequences at two locations can be selected by maintaining the cells under conditions that induce the marker gene for negative selection, and using the absence of the expression of the marker gene for negative selection as an index. When the marker gene for negative selection is a gene that kills cells (suicide gene), such a gene can be operably linked to an inducible promoter. Cells having no DNA region deletion can be removed by inducing the expression of the marker gene for negative selection through the action of an inducing factor on the inducible promoter. When the marker gene for negative selection is a visible marker gene, cells having no DNA region deletion can be removed by using the expressed visible marker gene as an index. When the marker gene for negative selection is a visible marker gene, the cells having the DNA region deletion can also be selected by using the absence of the expression of the visible marker gene as an index.

### <Cell having a deletion in DNA region>

A cell having a deletion of a target DNA region can be obtained by the method mentioned above. Thus, the present disclosure provides a cell having a deletion of a target DNA region. The target DNA region can include a gene essential for cell survival. Thus, the present disclosure provides a cell having a deletion of a target DNA region, wherein the target DNA region includes a gene essential for cell survival. The cell having a deletion of a target DNA region, wherein the target DNA region includes a gene essential for cell survival can have genomic DNA having an ectopic insert of the gene essential for survival. Thus, the present disclosure provides a cell having a deletion of a target DNA region, wherein the target DNA region includes a gene essential for cell survival, and the cell has genomic DNA having an ectopic insert of the gene essential for cell survival. The ectopic insertion location is not particularly limited to and can be a region where other genes are absent. The cells for use in the (a) and the (α) may be a cell having a deletion of a target DNA region, wherein the target DNA region includes a gene essential for cell survival, and the cell has genomic DNA having an ectopic insert of the gene essential for cell survival. As a result, by the (b) or the (β), the DNA region to be deleted can be further expanded, and the deletion can be extended to a subsequent gene that controls cell survival and/or proliferation (e.g., gene that positively controls cell survival and/or proliferation, for example, gene essential for cell survival and/or proliferation). The cell having a deletion of a target DNA region, obtained by the method mentioned above can have a proliferation rate or a survival rate of 50% or more, 60% or more, 70% or more, 80% or more, 90% or more, or 95% or more as compared with the cell before deletion introduction. In an aspect of the disclosure, in the cell having a deletion of a target DNA region, 20% or more, 30% or more, 40% or more, 50% or more, 60% or more, 70% or more, 80% or more, or 90% or more of the original genome is deleted.

### <Method for producing cell defective in DNA region>

The cell having a deletion of a target DNA region can be prepared by the method described above. Thus, the present disclosure provides a method for producing a cell having a deletion of a target DNA region, comprising carrying out the method of the present disclosure. The cells having the DNA region deletion can be selected on the basis of lack of the marker gene for negative selection.

### Examples

### Material and method

### Cell culture

HAP1 cells were cultured in Iscove's modified Dulbecco's medium (IMDM) supplemented with 10% (v/v) fetal bovine serum and 100 U/mL penicillin/streptomycin at 37°C in an atmosphere containing 5% CO₂. HCT116 cells were cultured under the same conditions as above except that McCoy's 5A medium was used.

### Prediction of essential gene based on past large-scale experiment

Essential genes of HAP1 cells were defined according to the following two criteria: (1) annotated as being essential in at least one of three reports (OGEE database¹, CRISPR screening², and Gene-trap screening³), and (2) transcribed in the HAP1 cells [Transcript per million > 0; the data can be obtained from Human Protein Atlas (www.proteinatlas.org/humancell)].

### Plasmid construction

For the construction of 24 gRNA/Cas9 plasmids (gpHY001-pJS067), gRNA sequences were designed using CRISPRdirect software⁴. gRNA oligonucleotides (Table 1) were annealed and ligated with pX330 (Addgene) linearized in accordance with a standard protocol⁵.

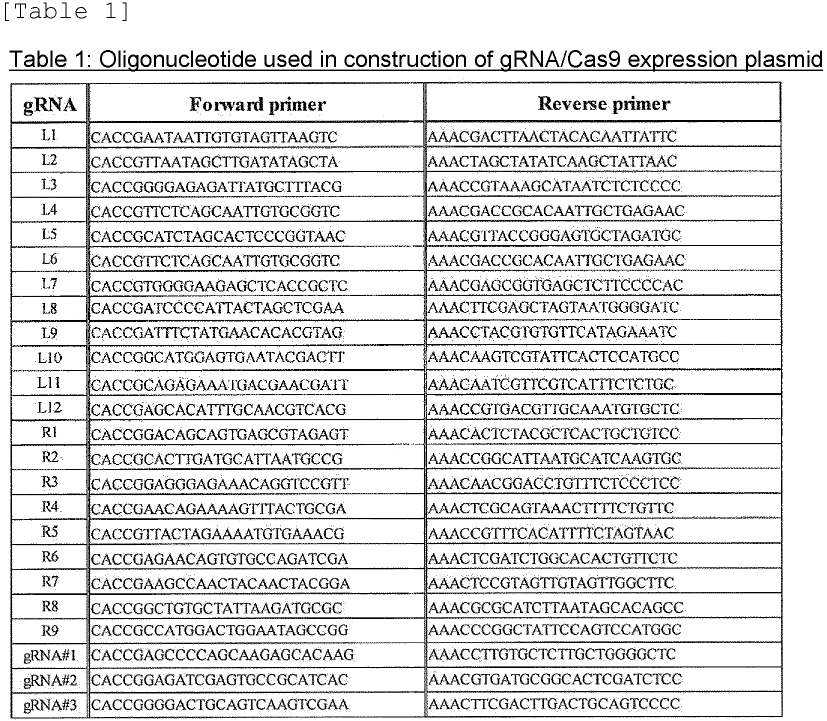

DH5 alpha *E. coli* cells were transformed with the ligated products, and plasmids were purified using EndoFree Plasmid mini Kit (Qiagen N.V.) in accordance with manufacturer's protocol.

pHY262 was constructed by inserting IRES-puro cassette to pWZ267 vector⁶, and further inserting a sequence called sgRNA (sg-A) site targeting pJS050 to 5' UTR of the IRES-puro cassette. TransforMax(TM) EPI300(TM) *E. coli* (Epicentre) was transformed with pHY262.

pHY263 was constructed by inserting IRES-GFP-2A-Puro cassette to pGEM(TM)-T Easy Vector (Promega Corp.).

pHY271 (for a thymidine kinase negative selection marker) was constructed by binding the amplification products shown in Tables 2-1 and 2-2 using Gibson Assembly. DH5 alpha *E. coli* cells were transformed with the Gibson Assembly product. Plasmids were purified using EndoFree Plasmid Midi Kit (Qiagen N.V.) in accordance with manufacturer's protocol.

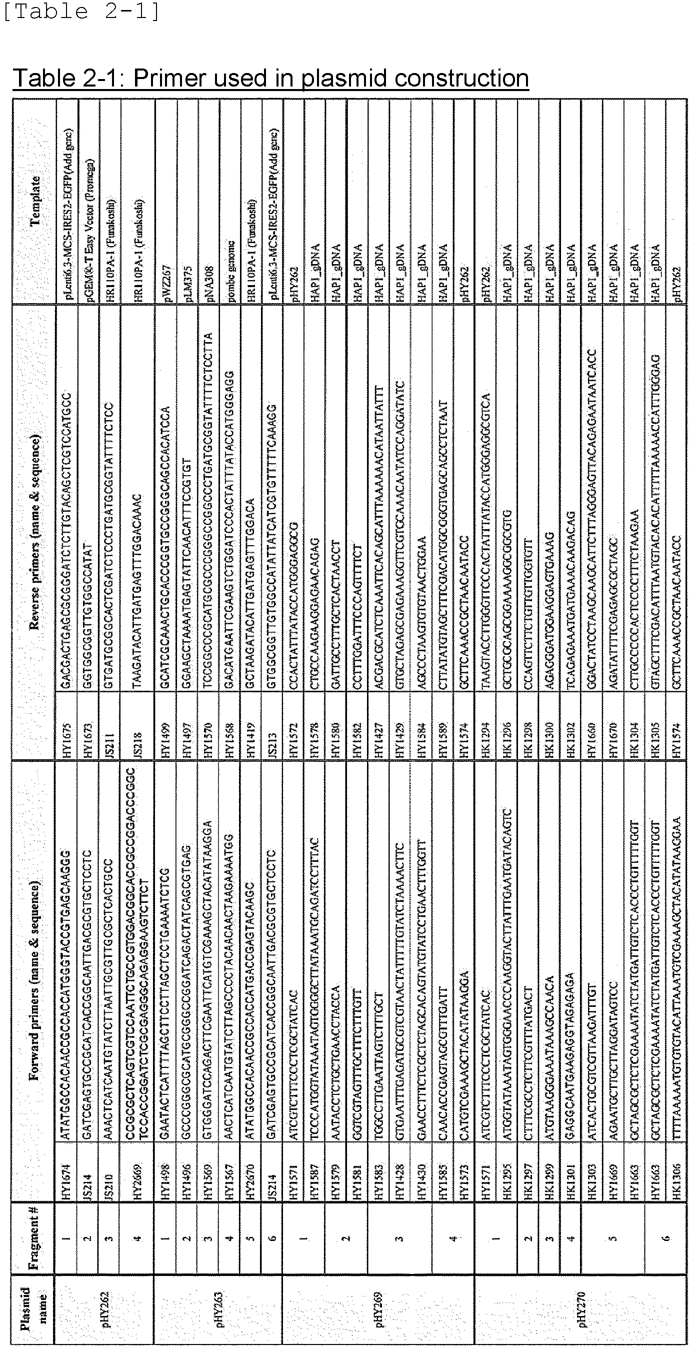

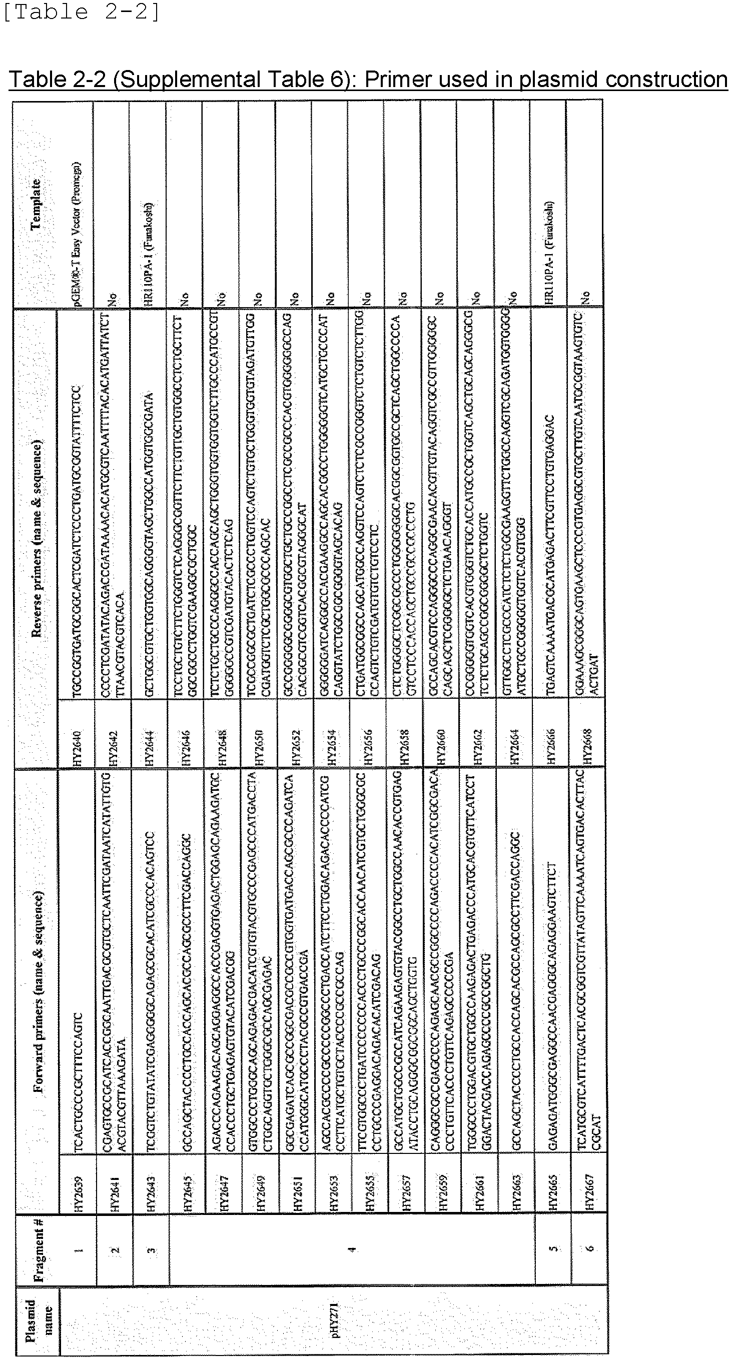

pHY269 (for RBMX2 gene locus cloning) and pHY270 (for MMGT1 gene locus cloning) were constructed. pHY262 (up to 100 ng) was linearized with BamHI and EcoRI (New England Biolabs Inc.), and yeast was co-transformed with this plasmid and up to 100 ng of a genome amplification product (Supplemental Table 6) that covered a RBMX2 or MMGT1 gene locus containing a 3-kb region upstream of a transcription start site and a 3-kb region downstream of a polyadenylation site. Each fragment had an overlap of up to 300 bp. The yeast transformation was performed in accordance with the previously described protocol⁷. Plasmids were extracted from yeast colonies and recovered into TransforMax(TM) EPI300(TM) *E. coli* (Epicentre) in accordance with the protocol⁸. The *E. coli* was cultured in LB using CopyControl(TM) Induction Solution (Epicentre), and plasmids were purified using EndoFree Plasmid Midi Kit (Qiagen N.V.) in accordance with manufacturer's protocol. In order to confirm the constructed plasmids, the purified plasmids were digested with suitable restriction enzymes and separated by agarose gel electrophoresis.

### Transfection

HAP1 cells were inoculated at a density of 6 × 10⁵ cells/well to a 12-well plate and incubated overnight. 50 µL of 2.5 M CaCl₂ containing 1.5 µg of DNA was prepared and mixed with 50 µL of 2 × BBS buffer (400 mM boric acid, 300 mM NaCl, and 5 mM EDTA). This solution was incubated at 25°C for 5 minutes, then mixed with 1 mL of IMDM, added to the cell cultures, and incubated for 4 to 8 hours. Then, the medium was aspirated, and the cells were washed twice with D-PBS (Nacalai Tesque, Inc., #14249-24). Then, fresh IMDM medium was added thereto and incubated at 37°C under 5% CO₂. HCT116 cells were inoculated at a density of 3 × 10⁵ cells/well to a 12-well plate and incubated overnight. FuGENE(TM) HD (Promega Corp.) transfection was performed at a ratio of 3:1 in accordance with manufacturer's instruction (FuGENE(TM) HD 3 µL Transfection Reagent per well: 900 ng DNA).

### Digital junction PCR

Each target region was subjected to 48 reactions in a nest PCR format. The first run of PCR employed 100 genomes (in the case of haploid human cells, 3.3 pg of genomic DNA) per reaction, and the second run of PCR employed a 1/100 dilution of the first PCR mixture as a template using 2 × Quick Taq HS DyeMix (Toyobo Co., Ltd.) and the primers described in Tables 3-1 and 3-2. Deletion efficiency (λ) was calculated using Poisson statistics: λ = -ln(1 - p) wherein λ is an average number of genomes having a deletion junction among the 100 genomes, and p is the ratio of positive reaction in the 48 runs of PCR⁹.

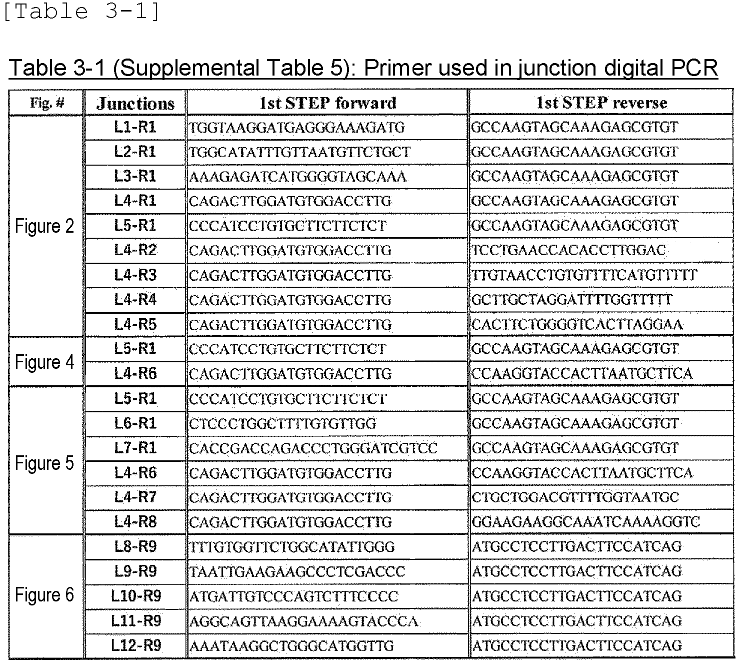

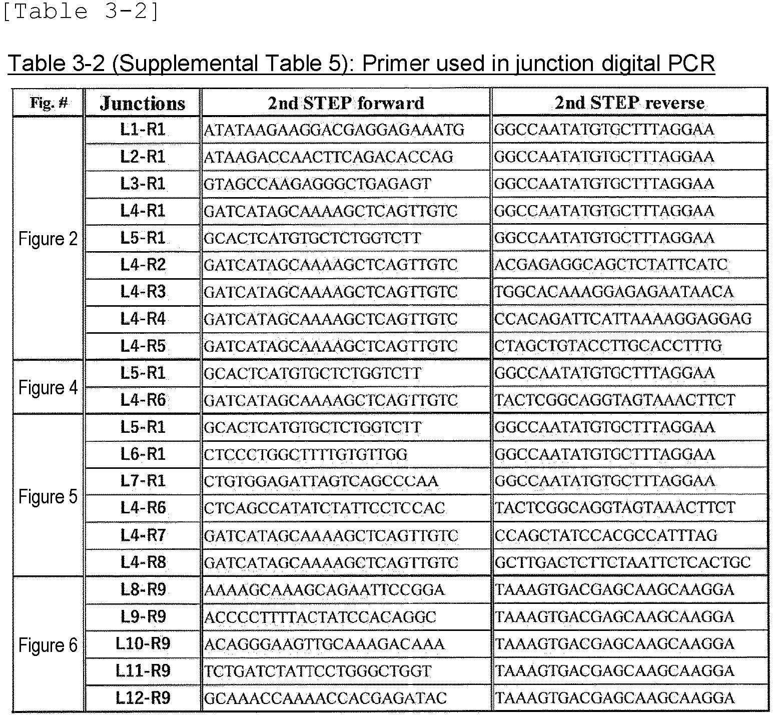

### Isolation of surviving colony after 6-TG selection

HAP1 cells were transfected with gRNA/Cas9 expression vectors, and then, up to 2,000 cells were treated with trypsin, reinoculated to a 10 cm dish, and cultured for 12 days with 5 µM 6-thioguanine (6-TG) (Sigma-Aldrich Co., LLC, A4882-100MG). Then, independent colonies on the 10 cm dish were picked up and separately developed into wells of a 24-well plate. Subsequently, some cells were developed in a 12-well plate for genomic DNA isolation, and the remaining individual clones were used as a frozen stock for further analysis.

### Colony formation assay after 6-TG selection

HAP1 or HCT116 was transfected with gRNA/Cas9 expression vectors, and then, up to 5 × 10⁴ cells were treated with trypsin, reinoculated to a 6 cm dish, and cultured for 9 days with 5 µM 6-TG (Sigma-Aldrich Co., LLC, A4882-100MG). Then, the medium was removed from the 6 cm dish, and EtBr solution (0.5% of 10 mg/mL ethidium bromide (Nacalai Tesque, Inc.) in 50% ethanol) was added to the cells and incubated at room temperature for 30 seconds. The EtBr solution was removed, and colonies were visualized with a UV illuminator¹⁰. The number and size of colonies stained were each quantified using "find maxima" and "analysis particle" functions of ImageJ software.

### Genomic DNA extraction

Cells cultured in a 12-well plate were collected into a 1.5 mL tube, resuspended in 118 µL of a breaking buffer (10 mM Tris-HCl (pH 8.0), 100 mM NaCl, 0.04% (w/v) SDS, 0.2 mg/mL proteinase K (FUJIFILM Wako Pure Chemical Corp.), and 2.5 mg/mL RNase A (Nippon Gene Co., Ltd.)), and incubated at 37°C for up to 18 hours. One volume of phenol/chloroform/isoamyl alcohol (25:24:1) was added thereto and rotated at 60 rpm for 30 minutes. After centrifugation at 12,000 rpm for 10 minutes, an aqueous layer as an upper layer was transferred to a fresh tube, and one volume of absolute isopropanol was added thereto. After centrifugation at 13,000 rpm for 5 minutes, pellets were washed with 300 µL of 70% ethanol and dried in air. Genomic DNA was dissolved in 100 µL of TE buffer solution and stored at -20°C.

### Detection of deletion junction and sequence determination

A junction having a deletion (deletion junction) was amplified from extracted genomic DNA in a nest PCR format using 2 × Quick Taq HS DyeMix (Toyobo Co., Ltd.) and the PCR primers described in Supplemental Table 5. The nucleotide sequences of all PCR products were determined using ABI PRISM BigDye Terminator Cycle Sequencing kit (Applied Biosystems Inc.) and ABI 3100 DNA sequencer (Figure 1d).

### Detection of gene deletion in deletion clone

For the detection of gene deletions, extracted genomic DNA was amplified using 2 x Quick Taq HS DyeMix (Toyobo Co., Ltd.) and the PCR primers described in Tables 4-1 and 4-2, and a nest PCR format was performed in order to confirm that a gene to be deleted was deleted from the genome. The obtained PCR products were analyzed by 1% agarose gel electrophoresis (Figure 3a).

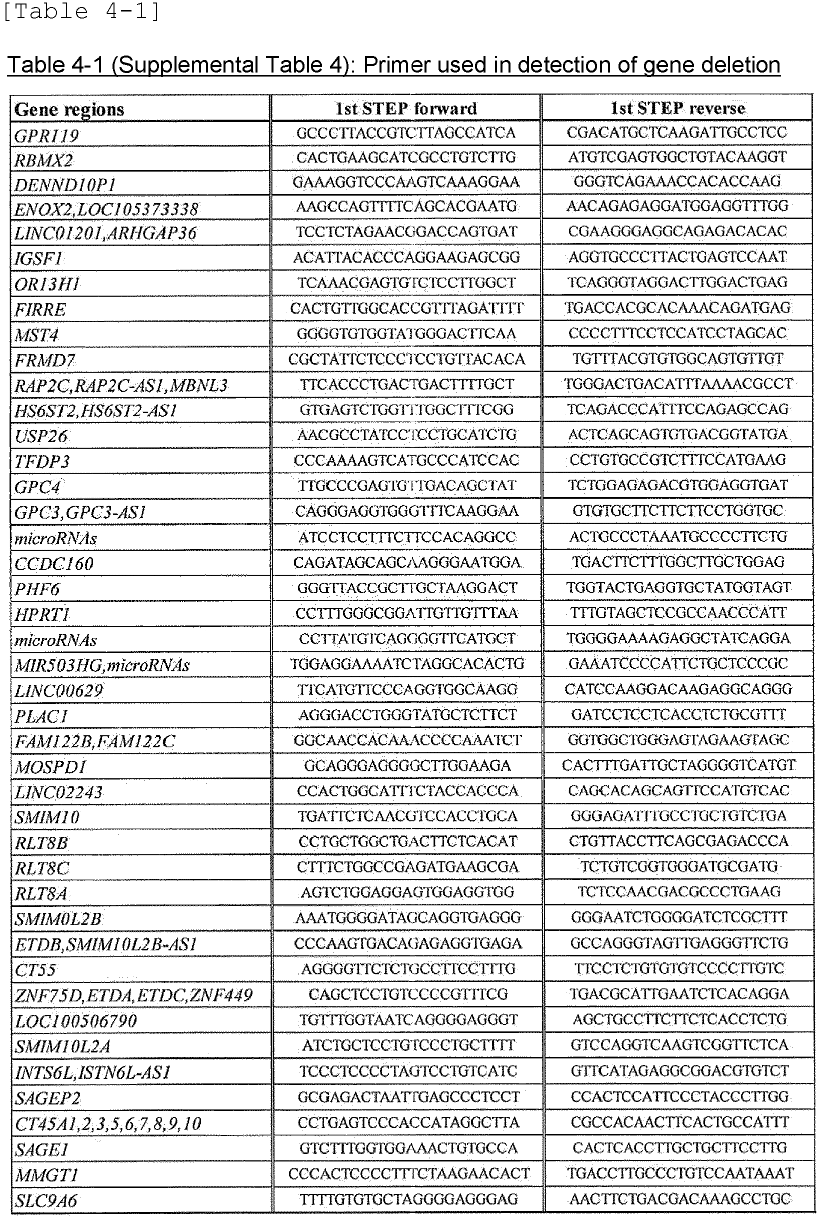

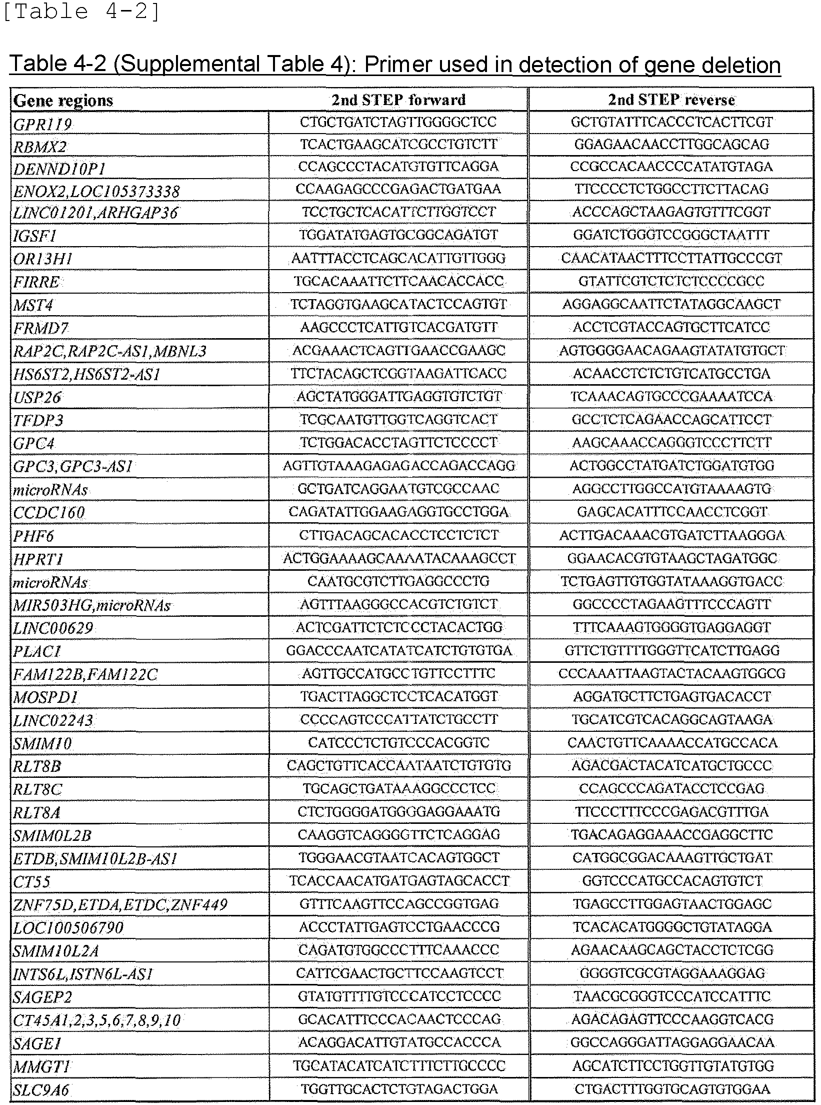

### Analysis of growth rate

Each HAP1 deletion clone or WT clone was mixed with hHY224 (in which IRES-GFP-2A-Puro cassette was integrated in 3' UTR of a GAPDH gene locus of HAP1 cells) and cultured in IMDM medium. The cells thus cultured for 2 days were recovered as "day_0" sample, and the remaining cells were further cultured for 16 days and recovered as "day_16" sample. GFP-positive cells and -negative cells were measured by flow cytometry. The ratio of the GFP-negative cells to the deletion clones was divided by the ratio of the GFP-negative cells to the WT clones to calculate a relative population. Flow cytometry analysis was conducted using EC800 flow cytometry analyzer (manufactured by Sony Biotechnology Inc.).

### Microarray analysis

Total RNA was extracted from HAP1 cells using RNeasy Mini RNA isolation kit (Qiagen N.V.). Microarray analysis was conducted using SurePrint G3 Human GE 8 x 60K Microarray (Agilent Technologies Inc.). Data analysis was conducted using Agilent Feature Extraction Software (Agilent Technologies Inc.). Only genes having a signal evaluation score = 2 in all wild-type clones or deletion clones were selected for data plots. P value < 0.05 and two-fold change > 1 were regarded as being significant.

### Transfer of essential genomic gene locus or IRES-GFP-2A-Puro cassette to GAPDH gene locus

HAP1 cells were co-transfected with pHY269 (for RBMX2 gene locus cloning), pHY270 (for MMGT1 gene locus cloning), or pHY263 (plasmid with IRES-GFP-2A-Puro cassette for preparing hHY224 cells) using pJS039 (gRNA expression vector targeting 3' UTR of a GAPDH region) and pJS050 (gRNA expression vector targeting the sgRNA (sg-A) site of pHY262). On 2 days after transfection, the cells were cultured for up to 10 days in IMDM medium supplemented with 1 ng/ml puromycin. Since a population of some HAP1 cells becomes diploid, only a haploid population was collected using SH800Z (Sony Biotechnology Inc.). The haploid cells were cultured for 7 days by expansion culture before the cells were cryopreserved for long-term preservation. The cell line was thawed and cultured for 5 days in a usual medium, followed by the application of MEGES thereto.

### Integration of TK marker into HCT116 genome

HCT116 was co-transfected with pHY271 using pJS067 (gRNA expression vector targeting a portion downstream of an OCRL region) and pJS050 (gRNA expression vector targeting the sg-A site of pHY271). The cells thus transfected for 2 days were cultured for up to 10 days in McCoy's 5A medium (containing 10% FBS, 1% penicillin/streptomycin, and 1 ng/µL puromycin). A single colony was expanded and cultured for another 7 days before the cells were cryopreserved for long-term preservation. The integration of the marker was tested by PCR using HY2223 and HY2224 primers (Supplemental Table 1). The cell stock was thawed and cultured for 7 days in a usual medium before the application of MEGES thereto.

### Analysis of DNA content

2 × 10⁶ cells were suspended in 1 mL of 4% PFA/PBS and incubated at 4°C for 10 minutes. Then, the cells were spun down at 500 g for 3 minutes. Cell pellets were resuspended in 70% EtOH and preserved at -20°C. For PI staining, the cells were spun down at 500 g for 3 minutes, resuspended in 500 µL of PI solution (D-PBS (Nacalai Tesque, Inc.), 50 µg/mL propidium iodide (FUJIFILM Wako Pure Chemical Corp.), and 0.25 mg/mL RNase A (Nippon Gene Co., Ltd.)), and then incubated at 37°C for 10 minutes. Flow cytometry analysis was conducted using EC800 flow cytometry analyzer (manufactured by Sony Biotechnology Inc.).

### Quantitative RT-qPCR

Total RNA was extracted from HAP1 cells using RNeasy Mini RNA isolation kit (Qiagen N.V.). 400 ng of RNA was reverse-transcribed using PrimeScript RT Reagent Kit with gDNA eraser (Takara Bio Inc.). Quantitative PCR was performed using TB Green(TM) Premix Ex Taq(TM) (Takara Bio Inc.). Each data point was a mean of three independent experiments performed in duplicate. In order to normalize variations in gene expression level, ACTB mRNA was used as an internal control. Primer sequences are shown in Table 5.

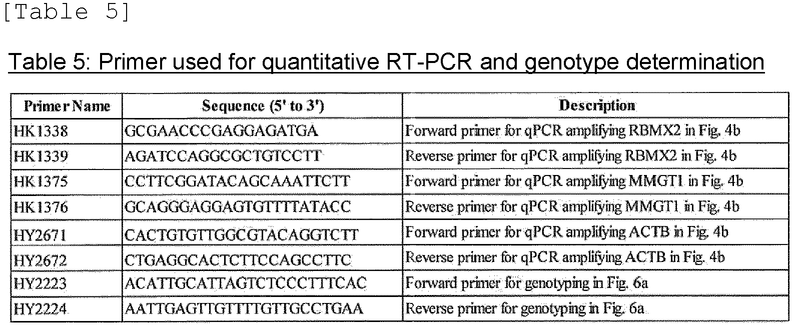

### [Results]

### Development of methodology for large-scale deletion in genome

In order to verify the concept of this experimental platform, an attempt was made to delete a genomic region on the chromosome X including hypoxanthine phosphoribosyl transferase 1 (HPRT1) gene of a human cell line HAP1 (Figure 1b). HPRT1 has been identified as an essential gene in particular human cells on the basis of the Online GEne Essentiality (OGEE) database [REF] and established as a negative selection marker with 6-TG (Nature Reviews Genetics, 6, 507-512 (2005)). If such a case holds true for HAP1 cells, HPRT1 cannot be used as a negative selection marker. The loss-of-function screening of HAP1 cells by Blomen's gene trapping³ or Mair's CRISPR screening² has indicated that HPRT1 is unnecessary for the proliferation of HAP1 cells. Deletions ascribable to a gRNA pair (e.g., gRNA L and gRNA R shown in Figure 1a) developed by us are a method different from gene knockout and may bring about different results. Accordingly, in order to examine whether a 0.51-Mb region including HPRT1 was essential, an approach was performed using a gRNA pair. Some cells were collected 2 days after introduction of a gRNA pair (R1 and L1) to HAP1 cells, and subjected to junction PCR. 14 out of 48 PCR reactions involving up to 100 genomes as templates caused amplification, and up to 0.3% of transfected HAP1 cells was found to have a target deletion between the R1 and L1 cleavage sites after exertion of an arbitrary proliferative effect. In a negative control brought about by gRNA R1 alone, no amplification occurred in any of the 48 reactions. This is explainable because an intact 0.51-Mb region was too long for amplification by junction PCR. In this way, subsequent 6-TG selection was predicted to provide a significant difference to the number of surviving cell colonies (Figure 1c). The step described above was able to remove 11 genes including HPRT1 and a nongenetic region within 0.51 Mb therein. As a result of analyzing the nucleotide sequence of genomic DNA extracted from 3 clones surviving after gRNA pair cleavage and 6-TG selection, it was confirmed that the target region was evidently deleted from the gene locus (Figure 1d). Our platform using HPRT1-6-TG negative selection was verified to be useful for obtaining HAP1 cell clones from which the nonessential region targeted by us was deleted.

### Expansion of deleted gene locus for genomic essential region detection

Next, whether our platform could be used in the identification of an essential gene region and/or an intergenic region was studied by expanding a deleted region in both the centromeric direction and the telomeric direction from HPRT1 (Figure 2). The first essential gene candidate from HPRT1 in the centromeric direction was ARHGAP36 on the basis of the OGEE database, and regions of 22 latently nonessential genes and intervening noncoding regions therebetween (corresponding to a 3.12 Mb region between the gRNA L1 and L2 sites in Figure 2b) reside between these genes. The next essential gene candidate was RBMX2. Three nonessential gene candidates reside between ARHGAP36 and RBMX2 (corresponding to a 0.62-Mb region between the gRNA L3 and L4 sites in Figure 2b), and a majority of this region is intergenic regions. Here, a region of approximately 4 Mb between gRNA L1 and L5 was divided into four sections (two essential gene candidate regions and two nonessential gene candidate clusters), and our platform was applied thereto to study whether any of essential gene candidates and/or broad intergenic regions were essential for the growth of HAP1 (Figure 2b).

On 2 days after deletion introduction, it was confirmed by digital junction PCR that five different gRNA pairs of L1, L2, L3, L4, or L5 with R1 achieved a deletion of the target region with equivalent efficiency (0.3-1.1%). It is to be noted that after 6-TG selection, several hundreds of colonies were formed with four out of the five gRNA pairs, whereas surviving cells were extremely lost by a deletion of a region between L4 and L5. This data reveals that the RBMX2 gene residing between the gRNA target sites L4 and L5 is important for normal proliferation of HAP1 and the region therebetween was deleted. This result is also consistent with the results of loss-of-function screening by Blomen's gene trapping³ or Mair's CRISPR screening², suggesting that the RBMX2 gene locus includes an essential element, whereas ARHGAP36 is absent in HAP1 cells. However, such conventional LOF screening can evaluate only the essence of one gene at once, whereas our platform provides new information on the whole Mb-scale genomic region including RBMX2, other 38 genes, and the dispersiveness of large-scale intergenic regions. Because of such high scalability, our platform was designated as MEGES.

The expansion of the deletion in the telomeric direction was continuously performed (Figure 2c). The first essential gene candidate supported by OGEE was SMIM10, and its essentiality had not been verified so far for HAP1 cells. The next OGEE candidate MMGT1 was suggested to be essential for HAP1 cells by Mair's CRISPR screening and however, suggested to be nonessential by Blomen's gene trapping. It should be noted that a 1.1-Mb region (between gRNA R1 and R4 sites) between HPRT1 and MMGT1 also included 30 nonessential gene candidates and intergenic sequence therebetween. MEGES utilized five different gRNA pairs of R2, R3, R4, or R5 with L4 to induce deletions. On 2 days after deletion introduction, deletions of all the target regions were efficiently induced, as verified by digital junction PCR. It was found that MMGT1 is essential, whereas the whole 5.48-Mb region between the gRNA L4 and R4 sites including SMIM10 can be deleted without largely influencing the growth of HAP1 cells.

### Characteristic evaluation of L4-R4 deletion clone

Four types of HAP1 cell clones (hHY131, 145, 148, and 149; L4-R4 deletion clones) were analyzed which were obtained by single clone development after MEGES using paired gRNA of R4 and L4. The obtained clones were confirmed to be haploid by use of flow cytometry using propidium iodo staining¹⁶. First, each individual gene locus positioned in a deletion region was genotyped by PCR. It is predicted that genomic fragments cleaved by gRNA pairs are randomly integrated into different chromosomal sites, and there may be a possibility that an essential gene within in this region was determined as a nonessential gene. Although 69 annotated genes reside between the gRNA R4 and L4 sites, most of the genes overlap or reside at very near positions. Accordingly, 39 gene loci that was able to cover all of the 69 gene loci were genotyped by PCR using genomic DNA extracted from the L4-R4 deletion clones. As shown in Figure 3a, it was found that in the L4-R4 deletion clones, all the tested gene loci were deleted from their genomes as compared with two clones (hHY153 and 154) of the original HAP1 that retained all the tested gene loci.

**[Table 6]**

| Table 6: Obtainment efficiency of cell having expanded deletion | | | | | | |
|---|---|---|---|---|---|---|
| Deletion region | R1 | L1-R1 | L2-R1 | L3-R1 | L4-R1 | L5-R1 |
| Length of deletion [kbp] | 0 | 508 | 3,628 | 3,725 | 4,347 | 4,379 |
| The number of clones analyzed | 6 | 12 | 12 | 12 | 24 | 6 |
| The number of clones having deletion | - | 11 | 12 | 12 | 24 | 0 |
| Efficiency [%] | - | 92* | 100 | 100 | 100 | 0 |

| Deletion region | L4 | L4-R2 | L4-R3 | L4-R4 | L4-R5 | |
|---|---|---|---|---|---|---|
| Length of deletion [kbp] | 0 | 4,542 | 4,577 | 5,476 | 5,498 | |
| The number of clones analyzed | 6 | 12 | 12 | 20 | 6 | |
| The number of clones having deletion | - | 12 | 12 | 20 | 0 | |
| Efficiency [%] | - | 100 | 100 | 100 | 0 | |

The data shown in Table 6 was obtained by analyzing the genotypes of the clones shown in Figure 2. Cell clones defective in the desired gene were counted as the number of cells having the deletion.

The influence of the 69 gene deletions on transcriptome was evaluated (Figure 3b). In this transcriptome analysis, RBMX2 and MMGT1 rarely had change in transcription level by a deletion of an adjacent 5.5-Mb region [Figure 3c], indicating that distant cis-regulatory elements of these genes are absent in this deletion region.

### Interchromosomal transplantation of essential element

The RBMX2 gene and the MMGT1 gene, if having no distant cis-regulatory element, may be handled as compact and independent gene units, as in many bacterial or yeast genes in genome design and synthesis. In order to verify this, an attempt was made to transplant both the genes to different chromosomal positions. The whole gene region ("rescue cassette") including a region of up to 3 kbp upstream of a transcription start site and a region of up to 3 kbp downstream of a polyadenylation (pA) site was cloned using BAC-YAC vector [Figure 4a]. In closed-ring pHY262 vector, a puromycin N-acetyl-transferase (PNAT) coding sequence resides between an IRES sequence and a pA signal. When HAP1 is transfected with the resulting rescue vector, the cells are co-transfected with two different gRNA/Cas9 vectors; one of the vectors is gRNA targeting 3' UTR of GAPDH gene on chromosome 12, and the other vector targets the immediate outside of the IRES sequence on the rescue gene locus vector¹⁵. Cells having an IRES-Puro-pA+ rescue cassette integrated in the GAPDH gene locus can be obtained by puromycin selection. Real-time RT-PCR verified that the mRNA level of RBMX2 or MMGT1 was increased by 1.5 to 2.0 times in the obtained cell clones [Figure 4b]. Subsequently, MEGES was performed to delete the RBMX2 or MMGT1 gene from the endogenous gene locus on the X chromosome. As a result, cells having the transplant exhibited more surviving cell colonies with equivalent gRNA-mediated deletion efficiency [Figure 4c]. These data demonstrated that both the gene loci can be transplanted to nonendogenous chromosomal sites and play a role as essential genes. The upstream and downstream 3-kb regions were also found to suffice for expressing both the genes at a sufficient level for normal cell proliferation. Particularly, by the interchromosomal transplantation of the essential gene, MEGES was able to further delete the genome of the HPRT1 gene locus and to reach a subsequent essential gene candidate [Figure 5].

### MEGES at non-HPRT1 gene locus

In order to expand the scope of MEGES, a test to delete a genomic region other than the HPRT1 gene locus was conducted in a different human cell line HCT116 using another negative selection marker. The HCT116 cells established from male patients with colon cancer have one X chromosome and therefore have one HPRT1 gene locus. A herpes simplex virus thymidine kinase (TK) expression cassette contains sequences encoding TK followed by 2A peptide flanked by PNAT, which are expressed under CMV promoter and SV40 pA signal [Figure 6a]. When a plasmid containing the TK expression cassette is used in co-transfection with two different gRNA/Cas9 vectors, one gRNA targets 3' UTR of a nonessential gene OCRL (Nucleic Acid Research, 2020, doi: 10.1093/nar/gkaa884) of HCT116 on the chromosome X, and another gRNA targets a portion immediately upstream of the CMV promoter on the TK expression cassette vector¹⁵. Cells selected because of puromycin resistance were confirmed by junction PCR to have the TK expression cassette at the targeted OCRL gene locus [Figures 6a and 6b]. Next, these cells were used in MEGES. All of five gRNA pairs (pairs of R9 with L8, L9, L10, L11, or L12) efficiently deleted a genomic region including the OCRL gene locus, as shown in deletion efficiency of 0.3 to 1.2 measured by junction digital PCR on 2 days after deletion introduction [Figure 6c]. When TK resided in a genome and was intracellularly expressed, ganciclovir selection which could induce impaired cell proliferation significantly resulted in many colonies for all the gRNA pairs except for the pair of L12 and R9, suggesting that the region between the gRNA target sites L11 and L12 included an element important for cell proliferation. In this way, the different selection markers and the different human cells used demonstrated that the basic strategy of MEGES is applicable to any chromosomal site.

### MEGES without negative selection

The screening and identification of genomic regions important for cell proliferation, and the isolation of cell clones having mega-based pair-scale deletions in target genomic regions were performed as described above, and the usefulness and advantages of MEGES were verified in order to further elucidate functional significance of the target regions. However, the screening of only essential regions requires preliminary integration of a negative selection marker. Furthermore, a problem of colony formation assay using negative selection was low throughput. Accordingly, we devised a modified version of MEGES involving digital junction PCR at a plurality of points in time instead of negative selection. When a deleted region is indispensable or important for cell proliferation, the ratio of cells having the deleted region decreases with the progression of cell culture. This change in the ratio can be evaluated by comparing deletion efficiency determined by use of digital junction PCR among a plurality of points in time after gRNA/Cas9 transfection. This strategy is referred to as digital junction PCR-based MEGES (dMEGES). In order to verify the concept, we applied dMEGES to a HPRT1 gene locus (Figure 7). We carried out digital junction PCR on 2 days and 17 days after cleavage with two gRNA pairs (R1-L4 and R1-L5) to verify expected results. The ratio of cells in which R1-L4 was cleaved was rarely changed from 2 days through 17 days after cleavage (from 1.8% to 2.0%), whereas the ratio of cells in which R1-L5 was cleaved drastically decreased from 1.0% to 0.2%. Here, cleavage with another gRNA pair of L13 with R1 which cleaved the inside of the RBMX2 gene locus was tested. As a result, the R1-L5 cleavage significantly decreased from 1.0% to 0.2%. This also brought about significant decrease in cell population having the deletion (from 2.1% to 0.6%). These data are consistent with the data of Figure 2b and indicate that a 4.35-Mb region between R1 and L4 was able to be deleted without influencing cell proliferation, whereas the R1-L5 region was an essential region of RBMX2 and therefore, was not able to be deleted.

A cell population having respective deletions in 22 regions on the X chromosome was prepared and analyzed in the same manner as above (Figure 8). As a result, as shown in Figure 8, the possibility was revealed that deletions of region 7 and region 16 are essential for cell proliferation or survival. Also, the possibility was revealed that regions **1,** 9 to 15, and 17 to 19 include a gene that positively influences cell proliferation or survival. Despite the deletions of 22 regions reported to be nonessential for survival by the previous CRISPR screening, the present invention enabled essential chromosomal regions to be determined with high sensitivity.

In this way, in the invention of the present application, MEGES can be carried out without introducing a negative selection marker beforehand. In this case, MEGES can cause large-scale genomic deletions and in addition, can determine a region where a gene essential for cell survival resides. When the gene essential for survival is determined, this gene may be integrated to another chromosome to further expand the genomic deletions. dMEGES can be a useful technique for the identification of a gene essential for cell survival and/or large-scale deletions in genomes.

### References for material and method

1. Chen, W.-H., Lu, G., Chen, X., Zhao, X.-M. & Bork, P. OGEE v2: an update of the online gene essentiality database with special focus on differentially essential genes in human cancer cell lines. Nucleic Acids Res 45, D940-D944 (2017).
2. Mair, B. et al. Essential Gene Profiles for Human Pluripotent Stem Cells Identify Uncharacterized Genes and Substrate Dependencies. Cell Reports 27, 599-615.e12 (2019).
3. Blomen, V. A. et al. Gene essentiality and synthetic lethality in haploid human cells. Science 350, 1092-1096 (2015).
4. Naito, Y., Hino, K., Bono, H. & Ui-Tei, K. CRISPRdirect: software for designing CRISPR/Cas guide RNA with reduced off-target sites. Bioinformatics 31, 1120-1123 (2015).
5. Ran, F. A. et al. Genome engineering using the CRISPR-Cas9 system. Nat Protoc 8, 2281-2308 (2013).
6. He, X. et al. Knock-in of large reporter genes in human cells via CRISPR/Cas9-induced homology-dependent and independent DNA repair. Nucleic Acids Res 44, e85 (2016).
7. Zhang, W. et al. Engineering the ribosomal DNA in a megabase synthetic chromosome. Science 355, (2017).
8. Muller, H. et al. Assembling Large DNA Segments in Yeast. in Gene Synthesis (ed. Peccoud, J.) vol. 852 133-150 (Humana Press, 2012).
9. Wu, Z. et al. Absolute quantification of DNA methylation using microfluidic chip-based digital PCR. Biosensors and Bioelectronics 96, 339-344 (2017).
10. Guda, K., Natale, L. & Markowitz, S. D. An improved method for staining cell colonies in clonogenic assays. Cytotechnology 54, 85-88 (2007).

### References regarding matters other than material and method

1. Xavier, J. C., Patil, K. R. & Rocha, I. Systems Biology Perspectives on Minimal and Simpler Cells. Microbiol Mol Biol Rev 78, 487-509 (2014).
2. Posfai, G. Emergent Properties of Reduced-Genome Escherichia coli. Science 312, 1044-1046 (2006).
3. Hutchison, C. A. et al. Design and synthesis of a minimal bacterial genome. Science 351, aad6253-aad6253 (2016).
4. Franchini, L. F. & Pollard, K. S. Human evolution: the non-coding revolution. BMC Biology 15, (2017).
5. Fesebotte, C. & Pritham, E. J. DNA Transposons and the Evolution of Eukaryotic Genomes. Annu Rev Genet 41, 331-368 (2007).
6. Keeling, P. J. & Palmer, J. D. Horizontal gene transfer in eukaryotic evolution. Nat Rev Genet 9, 605-618 (2008).
7. Wang, T. et al. Identification and characterization of essential genes in the human genome. Science 350, 1096-1101 (2015).
8. He, Z. et al. Highly efficient targeted chromosome deletions using CRISPR/Cas9: Highly Efficient Targeted Chromosome Deletions. Biotechnology and Bioengineering 112, 1060-1064 (2015).
9. Wang, L. et al. Large genomic fragment deletion and functional gene cassette knock-in via Cas9 protein mediated genome editing in one-cell rodent embryos. Sci Rep 5, (2015).
10. Zhang, L. et al. Large Genomic Fragment Deletions and Insertions in Mouse Using CRISPR/Cas9. PLOS ONE 10, e0120396 (2015).
11. Essletzbichler, P. et al. Megabase-scale deletion using CRISPR/Cas9 to generate a fully haploid human cell line. Genome Res 24, 2059-2065 (2014).
12. Zheng, Q. et al. Precise gene deletion and replacement using the CRISPR/Cas9 system in human cells. BioTechniques 57, (2014).
13. Canver, M. C. et al. Characterization of Genomic Deletion Efficiency Mediated by Clustered Regularly Interspaced Palindromic Repeats (CRISPR)/Cas9 Nuclease System in Mammalian Cells. J. Biol. Chem. 289, 21312-21324 (2014).
14. Lee, H. J., Kim, E. & Kim, J.-S. Targeted chromosomal deletions in human cells using zinc finger nucleases. Genome Res. 20, 81-89 (2010).
15. He, X. et al. Knock-in of large reporter genes in human cells via CRISPR/Cas9-induced homology-dependent and independent DNA repair. Nucleic Acids Res 44, e85 (2016).
16. Olbrich, T. et al. A p53-dependent response limits the viability of mammalian haploid cells. Proceedings of the National Academy of Sciences 114, 9367-9372 (2017).
17. Dixon, J. R. et al. Topological domains in mammalian genomes identified by analysis of chromatin interactions. Nature 485, 376-380 (2012).
18. Nora, E. P. et al. Spatial partitioning of the regulatory landscape of the X-inactivation centre. Nature 485, 381-385 (2012).
19. Lupiáñez, D. G. et al. Disruptions of Topological Chromatin Domains Cause Pathogenic Rewiring of Gene-Enhancer Interactions. Cell 161, 1012-1025 (2015).
20. Guo, Y. et al. CRISPR Inversion of CTCF Sites Alters Genome Topology and Enhancer/Promoter Function. Cell 162, 900-910 (2015).
21. Haarhuis, J. H. I. et al. The Cohesin Release Factor WAPL Restricts Chromatin Loop Extension. Cell 169, 693-707.e14 (2017).
22. Wang, Y. et al. The 3D Genome Browser: a web-based browser for visualizing 3D genome organization and long-range chromatin interactions. Genome Biol 19, 151 (2018).
23. Suzuki, K. et al. Highly efficient transient gene expression and gene targeting in primate embryonic stem cells with helper-dependent adenoviral vectors. Proc Natl Acad Sci U S A 105, 13781-13786 (2008).
24. Frank, S., Skryabin, B. V. & Greber, B. A modified TALEN-based system for robust generation of knock-out human pluripotent stem cell lines and disease models. BMC Genomics 14,773 (2013).
25. Moore, M. M., Parker, L., Huston, J., Harrington-Brock, K. & Dearfield, K. L. Comparison of mutagenicity results for nine compounds evaluated at the hgprt locus in the standard and suspension CHO assays. Mutagenesis 6, 77-85 (1991).
26. Chen, W.-H., Minguez, P., Lercher, M. J. & Bork, P. OGEE: an online gene essentiality database. Nucleic Acids Research 40, D901-D906 (2012).

## Claims

1. An *in vitro* method comprising:
(a) providing a cell population comprising isolated cells;
(b) allowing a sequence-specific nucleic acid cleaving molecule capable of sequence-specifically cleaving target sequences at two locations on genomic DNA to act on genomic DNA of cells in the cell population so that cleavage occurs in each of the target sequences at two locations on the genomic DNA, thereby causing a DNA region deletion in a region between the cleavage sites at two locations in at least some cells in the cell population; and
(c) then culturing the obtained cell population, and determining the influence of the DNA region deletion on cell proliferation or survival, wherein in the (c), the determination is performed by: determining a DNA region deletion efficiency as a ratio of cells having the deletion to the total cells contained in a suspension containing the cell population; and then comparing said determined deletion efficiency with a ratio of cells having the DNA region deletion in the cell population to the total cells contained in a suspension containing the cell population after culture, and
wherein cell screening is not carried out between the steps (b) and (c).

2. The method according to claim 1, wherein the ratio is determined by a counting technique of genomic DNA having the deletion and genomic DNA having no deletion, contained in the suspension.

3. The method according to claim 1 or 2, further comprising
(d) determining whether or not the deleted DNA region compared with control genomic DNA includes a gene that controls cell survival and/or proliferation on the basis of the presence or absence or a magnitude of the influence on cell survival and/or proliferation in the (c) as compared with a cell population used as a control.

4. The method according to claim 3, wherein the cell population used as a control is a cell population comprising cells having a deletion of a larger DNA region, a smaller DNA region, or a different DNA region by the (b).

5. The method according to claim 3 or 4, further comprising identifying at least one gene that controls cell survival and/or proliferation from genes residing in the deleted DNA region compared with control genomic DNA.

6. The method according to any one of claims 1 to 5, further comprising
(f) identifying at least one gene that controls cell survival and/or proliferation from genes residing in the deleted DNA region.

7. The method according to claim 5 or 6, further comprising
(g) ectopically introducing at least one gene that controls cell survival and/or proliferation to the genomic DNA having the DNA region deletion, the gene being operably linked to a control sequence.

8. The method according to any one of claims 1 to 7,
wherein a size of the DNA region to be deleted in the (b) is 0.5 Mbp or more.

9. The method according to any one of claims 1 to 8,
wherein a size of the DNA region to be deleted in the (b) is 1 Mbp or more.

## Patentansprüche

1. *In vitro* Verfahren, umfassend:
(a) Bereitstellen einer Zellpopulation, die isolierte Zellen umfasst;
(b) Zulassen, dass ein sequenzspezifisches Nukleinsäurespaltendes Molekül, das fähig ist, sequenzspezifisch Zielsequenzen an zwei Orten auf genomischer DNA zu spalten, auf die genomische DNA von Zellen in der Zellpopulation einwirkt, so dass eine Spaltung in jeder der Zielsequenzen an zwei Orten auf der genomischen DNA stattfindet, wodurch eine Deletion der DNA-Region ("DNA region deletion") in einem Bereich zwischen den Spaltstellen an zwei Orten in mindestens einigen Zellen in der Zellpopulation verursacht wird; und
(c) anschließendes Kultivieren der erhaltenen Zellpopulation und Bestimmen des Einflusses der Deletion der DNA-Region auf Zellproliferation oder Zellüberleben,
wobei in (c) die Bestimmung durchgeführt wird durch:
Bestimmen einer Deletionseffizienz der DNA Region("DNA region deletion efficiency") als ein Verhältnis der Zellen mit der Deletion zu den Gesamtzellen, die in einer die Zellpopulation enthaltenden Suspension enthalten sind; und anschließendes Vergleichen der ermittelten Deletionseffizienz mit einem Verhältnis von Zellen, die die Deletion der DNA-Region in der Zellpopulation aufweisen, zu den Gesamtzellen, die in der die Zellpopulation enthaltenden Suspension nach der Kultur enthalten sind, und
wobei zwischen den Schritten (b) und (c) kein Zell-Screening durchgeführt wird.

2. Verfahren gemäß Anspruch 1, wobei das Verhältnis durch eine Zähltechnik von genomischer DNA mit der Deletion und genomischer DNA ohne Deletion, die in der Suspension enthalten sind, bestimmt wird.

3. Verfahren gemäß Anspruch 1 oder 2, ferner umfassend (d)Bestimmen, ob die deletierte DNA-Region im Vergleich zur genomischen Kontroll-DNA ein Gen enthält, das das Zellüberleben und/oder die Zellproliferation kontrolliert oder nicht, basierend auf dem Vorhandensein oder Fehlen oder einem Ausmaß ("magnitude") des Einflusses auf das Zellüberleben und/oder die Zellproliferation in (c) im Vergleich zu einer als Kontrolle verwendeten Zellpopulation.

4. Verfahren gemäß Anspruch 3, wobei die als Kontrolle verwendete Zellpopulation eine Zellpopulation ist, die Zellen umfasst, die eine Deletion einer größeren DNA-Region, einer kleineren DNA-Region oder einer anderen DNA-Region durch (b) aufweisen.

5. Verfahren gemäß Anspruch 3 oder 4, ferner umfassend das Identifizieren mindestens eines Gens, das das Zellüberleben und/oder die Zellproliferation reguliert, aus Genen, die in der deletierten DNA-Region liegen, im Vergleich zu genomischer Kontroll-DNA.

6. Verfahren gemäß irgendeinem der Ansprüche 1 bis 5, ferner umfassend
(f) Identifizieren mindestens eines Gens, das das Zellüberleben und/oder die Zellproliferation steuert, aus Genen, die in der deletierten DNA-Region liegen.

7. Verfahren gemäß Anspruch 5 oder 6, das ferner umfasst (g) ektopisches Einführen mindestens eines Gens, das das Zellüberleben und/oder die Zellproliferation steuert, in die genomische DNA, die die Deletion der DNA-Region aufweist, wobei das Gen operativ mit einer Kontrollsequenz verbunden ist.

8. Verfahren gemäß irgendeinem der Ansprüche 1 bis 7, wobei die Größe der in (b) zu deletierenden DNA-Region 0,5 Mbp oder mehr ist.

9. Verfahren gemäß irgendeinem der Ansprüche 1 bis 8, wobei die Größe der in (b) zu deletierenden DNA-Region 1 Mbp oder mehr ist.

## Revendications

1. Procédé in vitro comprenant :
(a) le fait de se mettre à disposition une population de cellules comprenant des cellules isolées ;
(b) le fait de permettre à une molécule de clivage d'acide nucléique spécifique à une séquence, apte à cliver de manière spécifique à la séquence des séquences cibles en deux emplacements sur de l'ADN génomique, pour agir sur l'ADN génomique de cellules de la population de cellules, de telle sorte qu'un clivage se produise, dans chacune des séquences cibles, en deux emplacements sur l'ADN génomique, provoquant ainsi
une suppression de région d'ADN dans une région située entre les sites de clivage aux deux emplacements, dans au moins certaines cellules de la population de cellules ;
(c) puis le fait de mettre en culture la population de cellules obtenue, et de déterminer l'influence de la suppression de région d'ADN sur la prolifération ou survie cellulaires, dans lequel, dans l'étape (c), la détermination est effectuée par : le fait de déterminer une efficacité de suppression de région d'ADN en tant que rapport entre des cellules présentant la suppression et le nombre total de cellules contenues dans une suspension contenant la population de cellules ; puis le fait de comparer ladite efficacité de suppression déterminée avec un rapport entre des cellules présentant la suppression de région d'ADN dans la population de cellules et le nombre total de cellules contenues dans une suspension contenant la population de cellules après la culture, et
dans lequel un criblage de cellules n'est pas mis en œuvre entre les étapes (b) et (c),

2. Procédé selon la revendication 1, dans lequel le rapport est déterminé par une technique de comptage d'ADN génomique présentant la suppression et d'ADN génomique ne présentant pas de suppression, contenus dans la suspension.

3. Procédé selon la revendication 1 ou la revendication 2, comprenant en outre (d) le fait de déterminer si la région d'ADN supprimée, comparée à l'ADN génomique témoin, inclut un gène qui contrôle la survie et/ou la prolifération des cellules, sur la base de la présence ou de l'absence ou de l'ampleur de l'influence sur la survie et/ou la prolifération des cellules dans l'étape
(c) par comparaison avec une population de cellules utilisée comme témoin.

4. Procédé selon la revendication 3, dans lequel la population de cellules utilisée comme témoin est une population de cellules comprenant des cellules présentant, de par l'étape (b), une suppression à région d'ADN plus grande, à région d'ADN plus petite ou à région d'ADN différente.

5. Procédé selon la revendication 3 ou la revendication 4, comprenant en outre le fait d'identifier au moins un gène qui contrôle la survie et/ou la prolifération des cellules à partir de gènes résidant dans la région d'ADN supprimée, en comparaison avec l'ADN génomique témoin.

6. Procédé selon l'une quelconque des revendications 1 à 5, comprenant en outre (f) le fait d'identifier au moins un gène qui contrôle la survie et/ou la prolifération des cellules à partir de gènes résidant dans la région d'ADN supprimée.

7. Procédé selon la revendication 5 ou la revendication 6, comprenant en outre (g) le fait d'introduire de manière ectopique au moins un gène qui contrôle la survie et/ou la prolifération des cellules dans l'ADN génomique présentant la suppression de région d'ADN, le gène étant lié fonctionnellement à une séquence de contrôle.

8. Procédé selon l'une quelconque des revendications 1 à 7, dans lequel la taille de la région d'ADN à supprimer à l'étape (b) est supérieure ou égale à 0,5 Mbp.

9. Procédé selon l'une quelconque des revendications 1 à 8, dans lequel la taille de la région d'ADN à supprimer à l'étape (b) est supérieure ou égale à 1 Mbp.
